# EUROPEAN PATENT APPLICATION

(11) **EP 3 992 289 A1**
(43) Date of publication of application: **04.05.2022**
(21) Application number: 20425044.3
(22) Date of filing: 30.10.2020
(51) Int. Cl.: C12N 15/113, C12N 15/67, A61K 31/7088

(54) **FUNCTIONAL NUCLEIC ACID MOLECULES INCORPORATING PROTEIN BINDING DOMAIN**

(71) Applicant: Transine Therapeutics Limited, Cambridge CB23 6HJ (GB)
(72) Inventor: Takahashi, Hazuki, Royston, SG8 5B (GB); Toki, Naoko, Royston, SG8 5B (GB); Sharma, Harshita, Royston, SG8 5B (GB); Valentine, Matthew, Royston, SG8 5B (GB); Carninci, Piero, Royston, SG8 5B (GB)
(74) Representative: D Young & Co LLP

(57) **Abstract**

The invention relates to functional nucleic acid molecules which are targeted to an mRNA sequence to enhance protein translation, comprising at least one target determinant sequence, at least one RNA binding protein region and at least one regulatory sequence.

SEQUENCE LISTING

<110>  Transine Therapeutics Limited

<120>  FUNCTIONAL NUCLEIC ACID MOLECULES INCORPORATING PROTEIN BINDING
       DOMAINS

<130>  TSI-C-P2884EPp

<160>  78

<170>  PatentIn version 3.5

<210>  1
<211>  33
<212>  DNA
<213>  Homo sapiens

<400>  1
ccatggtggc gaccggtagc gctagcggat ctg                                    33


<210>  2
<211>  88
<212>  DNA
<213>  Homo sapiens

<400>  2
aagagggcac agccaccgag gatggtacag catggatgga tggtacgcta cagagactcg      60

ggagcccaac tgtgagtggc tgactggc                                          88


<210>  3
<211>  97
<212>  DNA
<213>  Homo sapiens

<400>  3
gagtgggtcc tgggcaaagc agggggtgca tcccagcctc agggaatagc acagcagagg      60

tctgttgatg catgcgagtg catgacctgc ttgccaa                                97


<210>  4
<211>  33
<212>  DNA
<213>  Homo sapiens

<400>  4
tgagtgggag aggggatgag acattccttt ctc                                    33

<210> 5
<211> 48
<212> DNA
<213> Homo sapiens

<400> 5
attactctgt gttggcggcg agctatctga cagccaacct tcccatac 48

<210> 6
<211> 118
<212> DNA
<213> Homo sapiens

<400> 6
ctagactcga gccatggtgg cgaccggtag cgctagcgga tctgacggtt cactagatgc 60

ggccgccact gtgctggata tctgcagaat tcgcccttca gtgctagagg aggtcaga 118

<210> 7
<211> 70
<212> DNA
<213> Homo sapiens

<400> 7
atgagcctgg aaagagggca cagccaccga ggatggtaca gcatggatgg atggtacgct 60

acagagactc 70

<210> 8
<211> 12
<212> DNA
<213> Homo sapiens

<400> 8
aagcatcatg gg 12

<210> 9
<211> 13
<212> RNA
<213> Artificial sequence

<220>
<223> Artificial sequence

<400> 9
aucugcagaa uuc 13

<210>  10
<211>  167
<212>  RNA
<213>  Mus musculus

<400>  10
cagugcuaga ggaggucaga agagggcauu ggauccccca gaacuggagu uauacgguaa      60

ccucguggug guugugaacc accaugugga uggauauuga guuccaaaca cugguccugu      120

gcaagagcau ccagugcucu uaagugcuga gccaucucuu uagcucc                   167


<210>  11
<211>  77
<212>  RNA
<213>  Mus musculus

<400>  11
   gaacuggagu uauacgguaa ccucguggug guugugaacc accaugugga uggauauuga      60
   guuccaaaca cuggucc                                                    77


<210>  12
<211>  29
<212>  RNA
<213>  Mus musculus

<400>  12
ccucguggug guugugaacc accaugugg                                        29


<210>  13
<211>  61
<212>  RNA
<213>  Mus musculus

<400>  13
guuauacggu aaccucgugg ugguugugaa ccaccaugug gauggauauu gaguuccaaa      60

c                                                                      61


<210>  14
<211>  98
<212>  RNA
<213>  Mus musculus

<400>  14
aucccccaga acuggaguua uacgguaacc ucgugguggu ugugaaccac cauguggaug      60

gauauugagu uccaaacacu gguccugugc aagagcau 98

<210> 15
<211> 129
<212> RNA
<213> Mus musculus

<400> 15
gaagagggca uuggaucccc cagaacugga guuauacggu aaccucgugg ugguugugaa 60

ccaccaugug gauggauauu gaguuccaaa cacugguccu gugcaagagc auccagugcu 120

cuuaagugc 129

<210> 16
<211> 183
<212> RNA
<213> Mus musculus

<400> 16
gggcagugcu agaggagguc agaagagggc auuggauccc ccagaacugg aguuauacgg 60

uaaccucgug gugguuguga accaccaugu ggauggauau ugaguuccaa acacuggucc 120

ugugcaagag cauccagugc ucuuaagugc ugagccaucu cuuuagcucc agucucuuaa 180

gcu 183

<210> 17
<211> 67
<212> RNA
<213> Mus musculus

<400> 17
gaacuggagu uauacgguaa ccucguggug guugugaacc accaugugga uggauauuga 60

guuccaa 67

<210> 18
<211> 107
<212> RNA
<213> Mus musculus

<400> 18
ggauccccca gaacuggagu uauacgguaa ccucguggug guugugaacc accaugugga 60

uggauauuga guuccaaaca cugguccugu gcaagagcau ccagugc 107

<210> 19
<211> 127
<212> RNA
<213> Mus musculus

<400> 19
agagggcauu ggauccccca gaacuggagu uauacgguaa ccucguggug guugugaacc 60

accaugugga uggauauuga guuccaaaca cugguccugu gcaagagcau ccagugcucu 120

uaagugc 127


<210> 20
<211> 38
<212> RNA
<213> Mus musculus

<400> 20
gguaaccucg uggugguugu gaaccaccau guggaugg 38


<210> 21
<211> 180
<212> RNA
<213> Artificial sequence

<220>
<223> Artificial sequence

<400> 21
cagugcuaga ggaggucaga agagggcauu ggauccccca gaacuggagu uauacgauaa 60

ccucguggug guugugaacc accaugugga uggauauuga guuccaaaca cugguccugu 120

gcaagagcau ccagugcucu uaagugcuga gccaucucuu uagcuccagu cucuuaagcu 180


<210> 22
<211> 180
<212> RNA
<213> Artificial sequence

<220>
<223> Artificial sequence

<400> 22
cagugcuaga ggaggucaga agagggcauu ggauccccca gaacuggagu uauacgcuaa 60

ccucguggug guugugaacc accaugugga uggauauuga guuccaaaca cugguccugu 120

gcaagagcau ccagugcucu uaagugcuga gccaucucuu uagcuccagu cucuuaagcu 180

<210> 23
<211> 180
<212> RNA
<213> Artificial sequence

<220>
<223> Artificial sequence

<400> 23
cagugcuaga ggaggucaga agagggcauu ggauccccca gaacuggcgu uauacgguaa 60

ccucguggug guugugaacc accaugugga uggauauuga guuccaaaca cugguccugu 120

gcaagagcau ccagugcucu uaagugcuga gccaucucuu uagcuccagu cucuuaagcu 180

<210> 24
<211> 180
<212> RNA
<213> Artificial sequence

<220>
<223> Artificial sequence

<400> 24
cagugcuaga ggaggucaga agagggcauu ggauccccca gaaguggagu uauacgguaa 60

ccucguggug guugugaacc accaugugga uggauauuga guuccaaaca cugcuccugu 120

gcaagagcau ccagugcucu uaagugcuga gccaucucuu uagcuccagu cucuuaagcu 180

<210> 25
<211> 180
<212> RNA
<213> Artificial sequence

<220>
<223> Artificial sequence

<400> 25
cagugcuaga ggaggucaga agagggcauu ggauccccca gauggugagu uauacgguaa 60

ccucguggug guugugaacc accaugugga uggauauuga guuccaaaca cgucaccugu 120

gcaagagcau ccagugcucu uaagugcuga gccaucucuu uagcuccagu cucuuaagcu 180

<210>  26
<211>  180
<212>  RNA
<213>  Artificial sequence

<220>
<223>  Artificial sequence

<400>  26
cagugcuaga ggaggucaga agagggcauu ggauccccca gaacugcacu auacgguaac      60

cucguggugg uugugaacca ccauguggau ggauauugag uuccaaauga gugguccugu      120

gcaagagcau ccagugcucu uaagugcuga gccaucucuu uagcuccagu cucuuaagcu      180


<210>  27
<211>  116
<212>  RNA
<213>  Artificial sequence

<220>
<223>  Artificial sequence

<400>  27
gggcauugga ucccccagaa cuggaguuau acgguaaccu cguggugguu gugaaccacc      60

auguggaugg auauugaguu ccaaacacug guccugugca agagcaucca gugcuc      116


<210>  28
<211>  77
<212>  RNA
<213>  Artificial sequence

<220>
<223>  Artificial sequence

<400>  28
ggacuggagu uauacgguaa ccucguggug guugugaacc accaugugga uggauauuga      60

guuccaaaca cugguccc                                                    77


<210>  29
<211>  77
<212>  RNA
<213>  Artificial sequence

<220>
<223>  Artificial sequence

<400>  29
gaacuggcgu uauacgguaa ccucguggug guugugaacc accaugugga uggauauuga      60

guuccaaaca cuggucc                                                     77


<210>  30
<211>  107
<212>  RNA
<213>  Mus musculus

<400>  30
gaugccuuag aaguggaguu aagaguugug agcugccguu uuuugguucu gggacucgaa      60

cucguuuccu cugauacuau caaccaccaa gccaucucuu cagcccc                   107


<210>  31
<211>  131
<212>  RNA
<213>  Mus musculus

<400>  31
gccagaagaa guugugggau ucccuggaac uggagcaacc aacaguuugu gugcaccaug      60

uggguaaugg gaaucgaacc uggguccucu auaagacugg ccagugcucu uaacuacuga     120

ggugcauuuc u                                                          131


<210>  32
<211>  187
<212>  RNA
<213>  Mus musculus

<400>  32
uuauuuuaaa uauaugagua uuucaccugc auaggcgcac aguacccaca gagacuagaa      60

gaggguggca gaucuccuga gacuggaguu aaugcuugug agcugccaug uggaugcugg     120

aaaucaaacc cagguccuuu ggaaggcagg caggugcucu uaaucaugga agcaucucuu     180

cagcucc                                                               187


<210>  33
<211>  131
<212>  RNA
<213>  Mus musculus

<400>  33
cagcgacauc agaagaggau auuggauccc auuacagaug guugaaggcc accaugucgu      60

ugcugggaau gaacucaaga ccucuggaag agcagucagu gcucuuaacc ucugagccau 120

cucuccagcc c 131

<210> 34
<211> 114
<212> RNA
<213> Mus musculus

<400> 34
auccccucca aagcucaaga ugguuguaag ccacccugug auugcuggga uuugaacuca 60

agaccuccgg aagagcaauu agugcucuua accgcugagc aaucucucca gccc 114

<210> 35
<211> 357
<212> RNA
<213> Mus musculus

<400> 35
gugcagugcu agaggagguc agaagagggc auuggauccc ccagaacugg aguuauacgg 60

uaaccucgug gugguuguga accaccaugu ggauggauau ugaguuccaa acacuggucc 120

ugugcaagag cauccagugc ucuuaagugc ugagccaucu cuuuagcucc uuauuuuaaa 180

uauaugagua uuucaccugc auaggcgcac aguacccaca gagacuagaa gaggguggca 240

gaucuccuga gacuggaguu aaugcuugug agcugccaug uggaugcugg aaaucaaacc 300

cagguccuuu ggaaggcagg caggugcucu uaaucaugga agcaucucuu cagcucc 357

<210> 36
<211> 532
<212> RNA
<213> Mus musculus

<400> 36
gugcagugcu agaggagguc agaagagggc auuggauccc ccagaacugg aguuauacgg 60

uaaccucgug gugguuguga accaccaugu ggauggauau ugaguuccaa acacuggucc 120

ugugcaagag cauccagugc ucuuaagugc ugagccaucu cuuuagcucc gugcgaauuc 180

ggugcagugc uagaggaggu cagaagaggg cauuggaucc cccagaacug gaguuauacg 240

guaaccucgu ggugguugug aaccaccaug uggauggaua uugaguucca aacacugguc 300

cugugcaaga gcauccagug cucuuaagug cugagccauc ucuuuagcuc cgugcgaauu 360

cggugcagug cuagaggagg ucagaagagg gcauuggauc ccccagaacu ggaguuauac 420

gguaaccucg uggugguugu gaaccaccau guggauggau auugaguucc aaacacuggu 480

ccugugcaag agcauccagu gcucuuaagu gcugagccau cucuuuagcu cc 532

<210> 37
<211> 228
<212> RNA
<213> Mus musculus

<400> 37
uuuuuuuaaa aauuuauuuu uauuuuaugu guaugagugu uuugccugca uguaugucug 60

uguaccacgu gcgugccugg ugcccgcgga ggccagaaga gggcgucgga uccccuggaa 120

cuggaguuac agaugguugu gagccgccau gugggugcug ggaaucgaac ccggguccuc 180

uggaagagca gccagugcuc uuaaccgcug agccaucucu ccagcccc 228

<210> 38
<211> 214
<212> RNA
<213> Mus musculus

<400> 38
uuuuuuuuac uuguauaggu guuuugccug cauguguauc uaucuaugua ccgaauaugu 60

uccugguauc cacagagacc aaaaguggau guuguaucuc cugaaauugg agucauagac 120

aguuaugagc ugccauuuga gugcuuggaa uagaacccag guccucuuaa agagcaucca 180

gugcucuuaa aaacugagac aucucuguag ccuc 214

<210> 39
<211> 200
<212> RNA
<213> Mus musculus

<400> 39
uuuauuuugc uuuauguguc ugaguguuug cuugaaugua ugucugugua ccacgccugu 60

accuugugcc uucagaguug agaggagggc auaggaucuc cuggaacugg aauugcaggu 120

gguugugagc cacccugugg guccugggga ccauacucca gcaagaacau caugugcucu 180

uaauuccuga gucuccaacc 200

<210> 40
<211> 214
<212> RNA
<213> Mus musculus

<400> 40
uuuauuuacu uaucuuuaug uguaugagug uguugucaga cuguuauguc ugugugucac 60

augcaugccu gcuguucaug gaguccagaa gagggcaucg gauccccugg aacuggaguu 120

acagaugagu ggccauguga auguuaagaa ccaaaccugg guccucugaa agagcagaca 180

augcucuuaa cuacugagcu gucucuccag cccc 214


<210> 41
<211> 205
<212> RNA
<213> Mus musculus

<400> 41
uuauuuuauu cguguaagug uuuugccagc aucuaugucu ucgcacuaug ugcaggucug 60

gugccugagg gguccagacg agagcacugg gucuccggga acuggaguua cagaucauug 120

ugagccacca ugugggugca gggaaucgaa ccugggaccu cuggaggagc agccacugcu 180

cuuaaccacu acacuauuuc uccag 205


<210> 42
<211> 121
<212> RNA
<213> Mus musculus

<400> 42
ucuguggacc acuguguaca gaagccugag aaggcuagca gauccccaga acuggaacug 60

ugagacgcug ugcuauggag gugcuaggaa cugaaaaugg auggguccuc ugcaagagca 120

g 121


<210> 43
<211> 191
<212> RNA
<213> Mus musculus

<400> 43
uuguuuuaau ugaauggcua uaggguguuu cuucuguaug uauaucuaug uuugguaccu 60

acagaggcau cagauccucu ggaacuguag uugcugacag uugugagcug ucauggggau 120

gcuggaauug aaccuggauc cuaugaaaga acagccagug uucuuaaccg cugagcuauc 180

ucuccaggcc c 191

<210> 44
<211> 205
<212> RNA
<213> Mus musculus

<400> 44
uuuuuuuuuu aauuuuaaaa aaaaagauuu uauuuauuua uuuuauauau gaugaguaca 60

cugucacucu uuucagacac ccuagaaaag gggggcauca gaucccauua cagaugguug 120

ugagccacau gguugcuggg aauugaccuc aggaccucug aaagagcagu cagugcucuc 180

aaccuuugag ucaucucucc agccc 205

<210> 45
<211> 190
<212> RNA
<213> Mus musculus

<400> 45
auguauaucu guaaugggac auacucacau acaugggcac gugaguauaa aaggccagaa 60

gagagcacug gacccucugg aguugagauu cuaagcaguu gugaaccauc ugauguaggu 120

gcugggaacu gaacuugggu ccuuugcuag agaaguaugu cucuuaacca cugagccgua 180

ucuccauccc 190

<210> 46
<211> 169
<212> RNA
<213> Mus musculus

<400> 46
uaaagauuua uucauuaagu acacuguagc uaucuucaga cgcaucagaa gagggcguca 60

gaucucuuua caggugguug ugagccacca ugugguugcu ggaauuugaa cucaggaccu 120

ucaaaagagc agucaguguu cuuaaccgcu gagccaucuc uccaacccc 169

<210> 47

<211>  159
<212>  RNA
<213>  Mus musculus

<400>  47
uuauuuauua uaaguacacu guagcugucu ucagacacaa caaaagaggg cgucagaucu      60

cauuacaggu gguugagcca ccaugugguu gcugggauuu gaacucagga ccuucagaac      120

agucagugcu cuuacccacu gagccagcga gccagcccc                             159


<210>  48
<211>  77
<212>  RNA
<213>  Artificial sequence

<220>
<223>  Artificial sequence

<400>  48
gaacuggagu uauacgguaa ccucguggug guugggaacc accaugugga uggauauuga      60

guuccaaaca cuggucc                                                     77


<210>  49
<211>  77
<212>  RNA
<213>  Artificial sequence

<220>
<223>  Artificial sequence

<400>  49
gaacuggagu uauacgguaa ccucguggug guucccaacc accaugugga uggauauuga      60

guuccaaaca cuggucc                                                     77


<210>  50
<211>  77
<212>  RNA
<213>  Artificial sequence

<220>
<223>  Artificial sequence

<400>  50
ggaccggagu uauacgguaa ccgcguggug guugugaacc accacgcgga uggauauuga      60

guuccaaaca ccggucc                                                     77

<210>  51
<211>  77
<212>  RNA
<213>  Artificial sequence

<220>
<223>  Artificial sequence

<400>  51
gaacuagagu uauacgguaa ccacauggug guugugaacc accaugugga uggauauuga      60

guuccaaaca cuaguuc                                                     77


<210>  52
<211>  206
<212>  RNA
<213>  Artificial sequence

<220>
<223>  Artificial sequence

<400>  52
uuauuuuaaa uauaugagua uuucaccugc auaggcgcac aguacccaca gagacuagaa      60

gaggguagua gauccccuag aacuggaguu auacgguaac cucguggugg uugugagcua     120

ccauguggau ggauacuggg aaucaaaccc agguccugug gaaggcaggc aggugcucuc     180

aagcacugag ccaucucuuc agcucc                                          206


<210>  53
<211>  206
<212>  RNA
<213>  Artificial sequence

<220>
<223>  Artificial sequence

<400>  53
uuauuuuaaa uauaugagua uuucaccugc auaggcgcac agugcucaag gagaucagaa      60

gagggcauca gaucuccuga gacuggaguu auacgguaac cucgugaugg uugugaacua     120

ccauguggau ggauauugag uuccaaacac agguccugug caagagcagc aggugcucuu     180

aagcacggaa ccaucucuuu agcucc                                          206

<210>  54
<211>  156
<212>  RNA
<213>  Artificial sequence

<220>
<223>  Artificial sequence

<400>  54
gaggcuagaa gaggguauca gauccccuga gacuggaguu auacgguaac cucguggugg      60

uugugagcca ccauguggau ggauacugag aaccaaaccc ugguccugug caagagcauc      120

aggugcucuu aagcacggaa ccaucucuuc agcucc                              156


<210>  55
<211>  68
<212>  RNA
<213>  Artificial sequence

<220>
<223>  Artificial sequence

<400>  55
guccugugca agagcaucga acucggugcu cuuaagcaca gaagccacca agccaucucu      60

ucagcccc                                                              68


<210>  56
<211>  110
<212>  RNA
<213>  Artificial sequence

<220>
<223>  Artificial sequence

<400>  56
cagugcuaga ggaggucaga agagggcauc ccccagccuc guggugguug ugaaccacca      60

uguggcugug caagagcaug cucuuaagug cugagccauc ucuuuagcuc                110


<210>  57
<211>  126
<212>  RNA
<213>  Artificial sequence

<220>
<223>  Artificial sequence

<400>  57
gagggcauug gaucccccag aacuggaguu auacgguaac cucguggugg uugugaacca      60

ccauguggau ggauauugag uuccaaacac ugguccugug caagagcauc cagugcucuu     120

aagugc                                                                126


<210>  58
<211>  89
<212>  RNA
<213>  Artificial sequence

<220>
<223>  Artificial sequence

<400>  58
ggauccccca gaacuggagu uauacgguaa ccucguggug guugugaacc accaugugga      60

uggauauuga guuccaaaca cugguccug                                       89


<210>  59
<211>  72
<212>  RNA
<213>  Artificial sequence

<220>
<223>  Artificial sequence

<400>  59
ugcuagagga ggucagaaga gggcauugga ugcaaaucca gugcucuuaa gugcugagcc      60

aucucuuuag cu                                                          72


<210>  60
<211>  94
<212>  RNA
<213>  Artificial sequence

<220>
<223>  Artificial sequence

<400>  60
gagggcauug gaucccccag aacuggaguu auacgguaac gauggauauu gaguuccaaa      60

cacugguccu gugcaagagc auccagugcu cuua                                  94


<210>  61
<211>  383

<212>  RNA
<213>  Hepatitis C virus

<400>  61
gccagccccc ugaugggggc gacacuccac caugaaucac uccccuguga ggaacuacug      60

ucuucacgca gaaagcgucu agccauggcg uuaguaugag ugucgugcag ccuccaggac     120

cccccucccc gggagagcca uaguggucug cggaaccggu gaguacaccg gaauugccag     180

gacgaccggg uccuuucuug gauaaacccg cucaaugccu ggagauuugg gcgugccccc     240

gcaagacugc uagccgagua guguuggguc gcgaaaggcc uugugguacu gccugauagg     300

gugcuugcga gugccccggg aggucucgua gaccgugcac caugagcacg aauccuaaac     360

cucaaagaaa aaccaaacgu aac                                             383


<210>  62
<211>  383
<212>  RNA
<213>  Hepatitis C virus

<400>  62
guuacguuug guuuuucuuu gagguuuagg auucgugcuc auggugcacg gucuacgaga      60

ccucccgggg cacucgcaag cacccuauca ggcaguacca caaggccuuu cgcgacccaa     120

cacuacucgg cuagcagucu ugcgggggca cgcccaaauc uccaggcauu gagcggguuu     180

auccaagaaa ggacccgguc guccuggcaa uuccggugua cucaccgguu ccgcagacca     240

cuauggcucu cccgggaggg gggguccugg aggcugcacg acacucauac uaacgccaug     300

gcuagacgcu uucugcguga agacaguagu uccucacagg ggagugauuc augguggagu     360

gucgccccca ucagggggcu ggc                                             383


<210>  63
<211>  312
<212>  RNA
<213>  Human poliovirus

<400>  63
augagucugg acaucccuca ccggugacgg ugguccaggc ugcguuggcg gccuaccuau      60

ggcuaacgcc augggacgcu aguugugaac aaggugugaa gagccuauug agcuacauaa     120

gaauccuccg gccccugaau gcggcuaauc ccaaccucgg agcagguggu cacaaaccag     180

ugauuggccu gucguaacgc gcaaguccgu ggcggaaccg acuacuuugg guguccgugu 240

uuccuuuuau uuuauugugg cugcuuaugg ugacaaucac agauuguuau cauaaagcga 300

auuggauugg cc 312

<210> 64
<211> 312
<212> RNA
<213> Human poliovirus

<400> 64
ggccaaucca auucgcuuua ugauaacaau cugugauugu caccauaagc agccacaaua 60

aaauaaaagg aaacacggac acccaaagua gucgguuccg ccacggacuu gcgcguuacg 120

acaggccaau cacugguuug ugaccaccug cuccgagguu gggauuagcc gcauucaggg 180

gccggaggau ucuuauguag cucaauaggc ucuucacacc uuguucacaa cuagcguccc 240

auggcguuag ccauagguag gccgccaacg cagccuggac caccgucacc ggugagggau 300

guccagacuc au 312

<210> 65
<211> 576
<212> RNA
<213> Encephalomyocarditis virus

<400> 65
cccccccucu cccucccccc ccccuaacgu uacuggccga agccgcuugg aauaaggccg 60

gugugcguuu gucuauaugu uauuuuccac cauauugccg ucuuuuggca augugagggc 120

ccggaaaccu ggcccugucu ucuugacgag cauuccuagg ggucuuuccc cucucgccaa 180

aggaaugcaa ggucuguuga augucgugaa ggaagcaguu ccucuggaag cuucuugaag 240

acaaacaacg ucuguagcga cccuuugcag gcagcggaac cccccaccug gcgacaggug 300

ccucugcggc caaaagccac guguauaaga uacaccugca aaggcggcac aaccccagug 360

ccacguugug aguuggauag uuguggaaag agucaaaugg cucuccucaa gcguauucaa 420

caaggggcug aaggaugccc agaagguacc ccauuguaug ggaucugauc uggggccucg 480

gugcacaugc uuuacaugug uuuagucgag guuaaaaaac gucuaggccc cccgaaccac 540

ggggacgugg uuuuccuuug aaaaacacga ugauaa 576

<210>  66
<211>  576
<212>  RNA
<213>  Encephalomyocarditis virus

<400>  66
uuaucaucgu guuuuucaaa ggaaaaccac guccccgugg uucggggggc cuagacguuu      60

uuuaaccucg acuaaacaca uguaaagcau gugcaccgag gccccagauc agaucccaua      120

caauggggua ccuucugggc auccuucagc cccuuguuga auacgcuuga ggagagccau      180

uugacucuuu ccacaacuau ccaacucaca acguggcacu gggguugugc cgccuuugca      240

gguguaucuu auacacgugg cuuuuggccg cagaggcacc ugucgccagg ugggggguuc      300

cgcugccugc aaagggucgc uacagacguu guuugucuuc aagaagcuuc cagaggaacu      360

gcuuccuuca cgacauucaa cagaccuugc auuccuuugg cgagagggga aagaccccua      420

ggaaugcucg ucaagaagac agggccaggu uuccgggccc ucacauugcc aaaagacggc      480

aauauggugg aaaauaacau auagacaaac gcacaccggc cuuauuccaa gcggcuucgg      540

ccaguaacgu uagggggggg ggagggagag gggggg                              576


<210>  67
<211>  192
<212>  RNA
<213>  Cricket paralysis virus

<400>  67
aaagcaaaaa ugugaucuug cuuguaaaua caauuuugag agguuaauaa auuacaagua      60

gugcuauuuu uguauuuagg uuagcuauuu agcuuuacgu uccaggaugc cuaguggcag      120

ccccacaaua uccaggaagc ccucucugcg guuuuucaga uuagguaguc gaaaaaccua      180

agaaauuuac cu                                                        192


<210>  68
<211>  192
<212>  RNA
<213>  Cricket paralysis virus

<400>  68
agguaaauuu cuuagguuuu ucgacuaccu aaucugaaaa accgcagaga gggcuuccug      60

gauauugugg ggcugccacu aggcauccug gaacguaaag cuaaauagcu aaccuaaaua      120

caaaaauagc acuacuugua auuuauuaac cucucaaaau uguauuuaca agcaagauca 180

cauuuuugcu uu 192

<210> 69
<211> 231
<212> RNA
<213> Homo sapiens

<400> 69
cagagaucca ggggaggcgc cugugaggcc cggaccugcc ccggggcgaa ggguaugugg 60

cgagacagag cccugcaccc cuaauucccg guggaaaacu ccuguugccg uuucccucca 120

ccggccugga gucucccagu cuugucccgg cagugccgcc cuccccacua agaccuaggc 180

gcaaaggcuu ggcucauggu ugacagcuca gagagagaaa gaucugaggg a 231

<210> 70
<211> 231
<212> RNA
<213> Homo sapiens

<400> 70
ucccucagau cuuucucucu cugagcuguc aaccaugagc caagccuuug cgccuagguc 60

uuagugggga gggcggcacu gccgggacaa gacugggaga cuccaggccg guggagggaa 120

acggcaacag gaguuuucca ccgggaauua ggggugcagg gcucugucuc gccacauacc 180

cuucgccccg gggcaggucc gggccucaca ggcgccuccc cuggaucucu g 231

<210> 71
<211> 460
<212> RNA
<213> Homo sapiens

<400> 71
acuuuuggug ggcauuuaaa aaugugugug uauguguaua uauguaugug uauguaugug 60

uauauaugua uauguaugua uguaucgcgu guaugugugu auguaugcau guguauguau 120

guauaugcau guauguguau guguauauau guaugugugu guauguauau guguguguau 180

guguaugugu guguguaugu guguguguau guauguaugu auguauaugu auuauacaca 240

uauacacaua uugguuuuuu uaaucauuug agaguuaguu gaagauaaaa acccaucacc 300

ccuaaaugua uuccaaagaa uaagaacauu guuuuauaca uagcacacuu aacaaaauca 360

agaaauuuaa cauuaauaca guacuguuac cuaauccgua gucgauuuuc aaauuuuguc 420

aguuguucca auaauguccu uuauauauuc cccgcccagc 460

<210> 72
<211> 460
<212> RNA
<213> Homo sapiens

<400> 72
gcugggcggg gaauauauaa aggacauuau uggaacaacu gacaaaauuu gaaaaucgac 60

uacggauuag guaacaguac uguauuaaug uuaaauuucu ugauuuuguu aagugugcua 120

uguauaaaac aauguucuua uucuuuggaa uacauuuagg ggugaugggu uuuuaucuuc 180

aacuaacucu caaaugauua aaaaaaccaa uauguguaua uguguauaau acauauacau 240

acauacauac auacacacac acauacacac acacauacac auacacacac auauacauac 300

acacacauac auauauacac auacacauac augcauauac auacauacac augcauacau 360

acacacauac acgcgauaca uacauacaua uacauauaua cacauacaua cacauacaua 420

uauacacaua cacacacauu uuuaaaugcc caccaaaagu 460

<210> 73
<211> 395
<212> RNA
<213> Homo sapiens

<400> 73
aauuccagcg agaggcagag ggagcgagcg ggcggccggc uaggguggaa gagccgggcg 60

agcagagcug cgcugcgggc guccugggaa gggagauccg gagcgaauag ggggcuucgc 120

cucuggccca gcccucccgc uugauccccc aggccagcgg uccgcaaccc uugccgcauc 180

cacgaaacuu ugcccauagc agcgggcggg cacuuugcac uggaacuuac aacacccgag 240

caaggacgcg acucucccga cgcggggagg cuauucugcc cauuugggga cacuuccccg 300

ccgcugccag gacccgcuuc ucugaaaggc ucuccuugca gcugcuuaga cgcuggauuu 360

uuuucgggua guggaaaacc agcagccucc cgcga 395

<210> 74

<211>  395
<212>  RNA
<213>  Homo sapiens

<400>  74
ucgcgggagg cugcugguuu uccacuaccc gaaaaaaauc cagcgucuaa gcagcugcaa      60

ggagagccuu ucagagaagc ggguccuggc agcggcgggg aagugucccc aaaugggcag      120

aauagccucc ccgcgucggg agagucgcgu ccuugcucgg guguuguaag uuccagugca      180

aagugcccgc ccgcugcuau gggcaaaguu ucguggaugc ggcaaggguu gcggaccgcu      240

ggccuggggg aucaagcggg agggcugggc cagaggcgaa gcccccuauu cgcuccggau      300

cucccuuccc aggacgcccg cagcgcagcu cugcucgccc ggcucuucca cccuagccgg      360

ccgcccgcuc gcucccucug ccucucgcug gaauu                                395


<210>  75
<211>  48
<212>  RNA
<213>  Homo sapiens

<400>  75
gggcacuuug cacuggaacu uacaacaccc gagcaaggac gcgacucu                  48


<210>  76
<211>  48
<212>  RNA
<213>  Homo sapiens

<400>  76
agagucgcgu ccuugcucgg guguuguaag uuccagugca aagugccc                  48


<210>  77
<211>  71
<212>  RNA
<213>  Homo sapiens

<400>  77
guacugacau cguagaugga aaucauaaac ugacucuugg uuugauuugg aauauaaucc      60

uccacuggca g                                                          71


<210>  78
<211>  71
<212>  RNA

<213>  Homo sapiens

<400>  78
cugccagugg aggauuauau uccaaaucaa accaagaguc aguuuaugau uuccaucuac      60

gaugucagua c                                                          71

## Description

### FIELD OF THE INVENTION

The present invention relates to functional nucleic acid molecules, particularly functional nucleic acid molecules, which have been modified to include specified protein binding domains. The functional nucleic acid molecules find particular use in upregulating target mRNA expression.

### BACKGROUND OF THE INVENTION

With the development of genomics technologies, it became widely recognized that an emerging class of long non-coding RNAs (IncRNAs), which constitute the majority of types of transcripts and do not encode proteins, play key regulatory roles in the physiology of normal cells, as well as in the development of diseases including cancer and neurodegenerative diseases.

The discovery of increasing numbers of functional IncRNAs has prompted novel therapeutic applications, including the treatment of human genetic diseases. A new class of long non-coding RNAs (IncRNAs), known as SINEUPs, were previously described to be able to selectively enhance their targets' translation. SINEUP activity relies on the combination of two domains: the overlapping region, or binding domain (BD), that confers specificity, and an embedded inverted SINE B2 element, or effector domain (ED), enhancing target mRNA translation. WO 2012/133947 and WO 2019/150346 disclose functional nucleic acid molecules including SINEUPs. Another class of IncRNAs that use effector domains comprising an internal ribosome entry site (IRES) sequence to provide trans-acting functional nucleic acid molecules are described in WO 2019/058304.

### SUMMARY OF THE INVENTION

According to a first aspect of the invention, there is provided a functional nucleic acid molecule comprising:
(a) at least one target determinant sequence comprising a sequence reverse complementary to a target mRNA sequence for which protein translation is to be enhanced;
(b) at least one sequence encoding an RNA binding protein region; and
(c) at least one regulatory sequence comprising a SINE B2 element or a functionally active fragment of a SINE B2 element or an internal ribosome entry site (IRES) sequence or an IRES derived sequence.

According to a further aspect of the invention, there is provided a DNA molecule encoding the functional nucleic acid molecule as defined herein.

According to a further aspect of the invention, there is provided an expression vector comprising the functional nucleic acid molecule or the DNA molecule as defined herein.

According to a further aspect of the invention, there is provided a composition comprising the functional nucleic acid molecule, the DNA molecule or the expression vector as defined herein.

According to a further aspect of the invention, there is provided a method for increasing the protein synthesis efficiency of a target in a cell comprising administering the functional nucleic acid molecule or the composition as defined herein, to the cell.

### BRIEF DESCRIPTION OF THE FIGURES

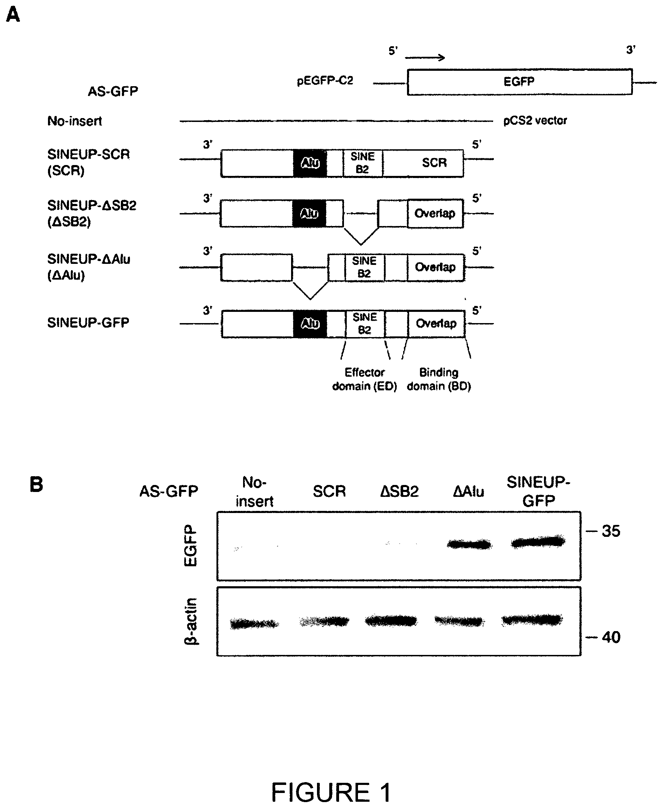

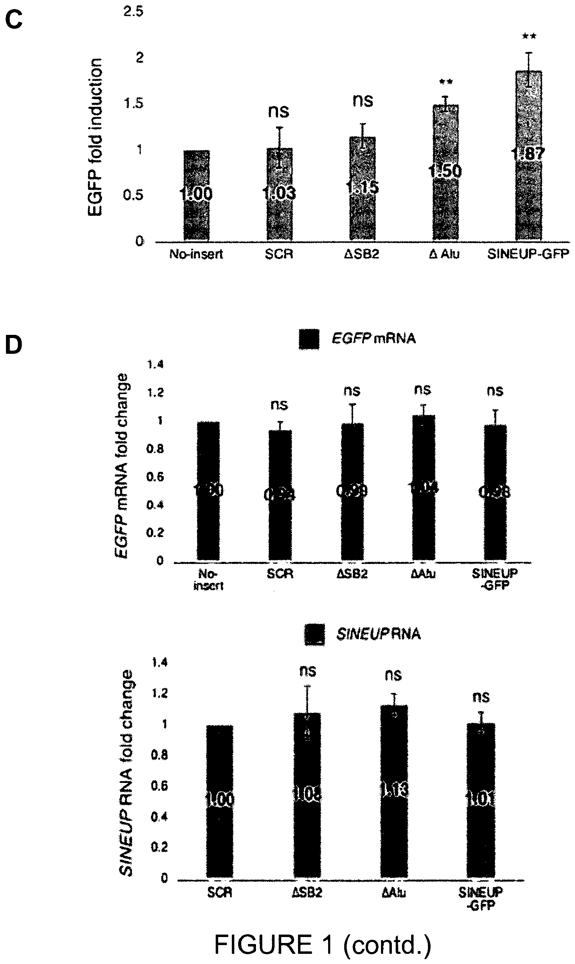

**FIGURE 1****: Enhancement of EGFP level by synthetic SINEUP-GFP.** A) Schematic representation of the SINEUP constructs used in this study. SINEUP-GFP contains the overlapping region with EGFP (binding domain, BD) and SINEB2 element (effector domain, ED). Domain deletion mutants constructed from SINEUP-GFP are shown: SINEUP-SCR (SCR) contains a scrambled sequence instead of the EGFP BD; SINEUP-delta SB2 (ΔSB2) has a deleted SINEB2 element; and SINEUP-delta Alu (ΔAlu) has a deleted Alu repeat element. B) Translational up-regulation of EGFP by co-transfection of EGFP and SINEUP expression vectors. Western blotting image showing the effect of SINEUPs on the EGFP level; the result shown is representative of at least three independent experiments. C) Quantification of the up-regulation of EGFP by co-transfection with EGFP and SINEUP vectors. **p < 0.01, ns: not significant, by Student's t-test. Data are means ± SD from at least 3 independent experiments. D) Quantification of the EGFP mRNA and SINEUP RNA levels following co-transfection with EGFP and SINEUP expression vectors. Data are means ± SD from at least 3 independent experiments.

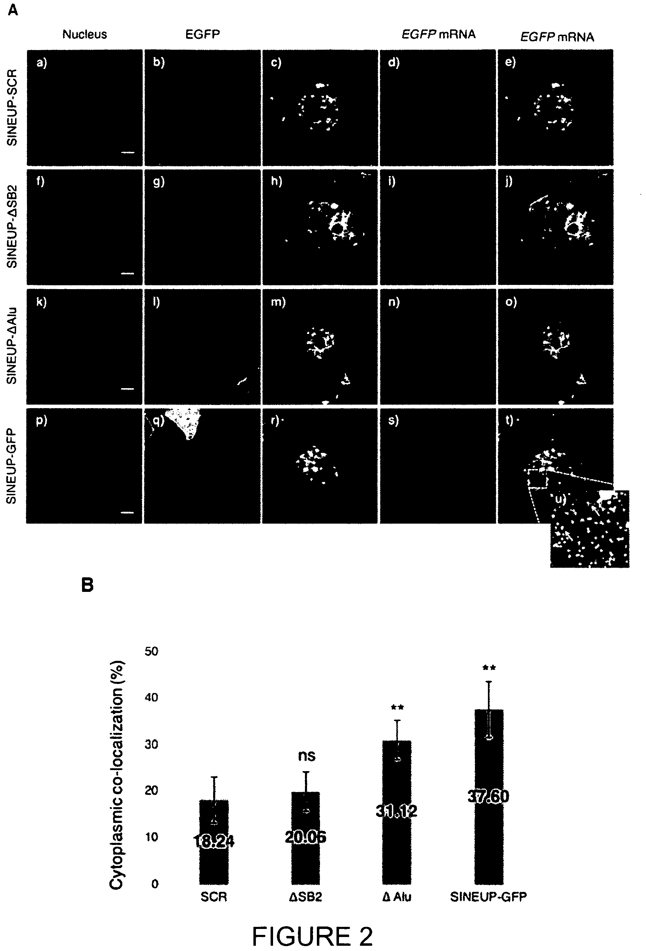

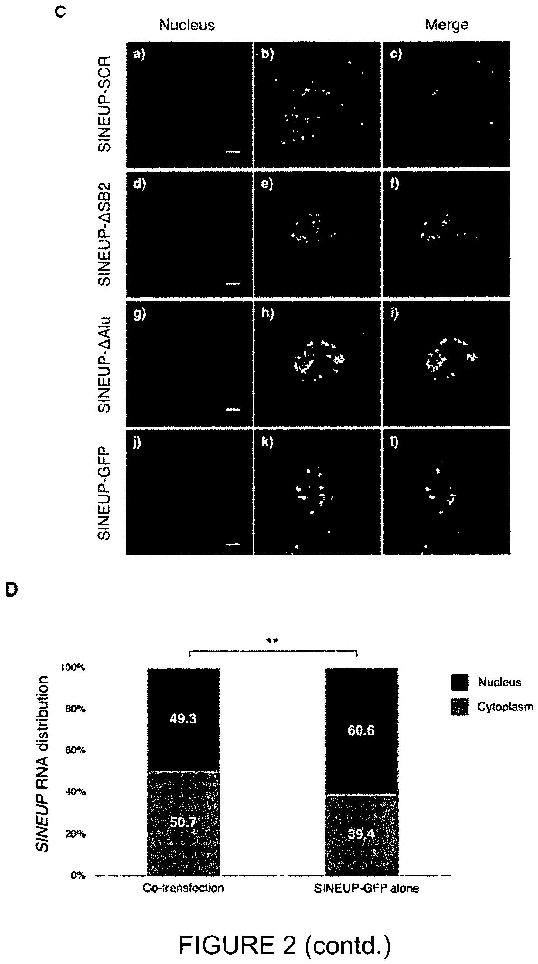

**FIGURE 2****: Co-localization of SINEUP-GFP RNAs with EGFP mRNAs in the cytoplasm.** A) Subcellular localization of SINEUP RNAs and EGFP mRNAs. Bars indicate 5 µm. B) Comparison of the percentage co-localization of EGFP mRNAs and SINEUP RNAs in the cytoplasm. Data are means ± SD of at least 20 independent cell images. **p < 0.01, ns: not significant by Student's t-test. C) Subcellular distribution of SINEUP RNAs following transfection with SINEUP expression vectors alone. Bars indicate 5 µm. D) Quantitative comparison of the distribution of SINEUP-GFP RNA in the presence and absence of EGFP mRNA. The ratios of detected spots in the nucleus and cytoplasm were compared between co-transfection of SINEUP-GFP and EGFP vectors, and transfection of SINEUP-GFP vector alone. Data were collected from at least 10 independent cell images. **p < 0.01 by Student's t-test.

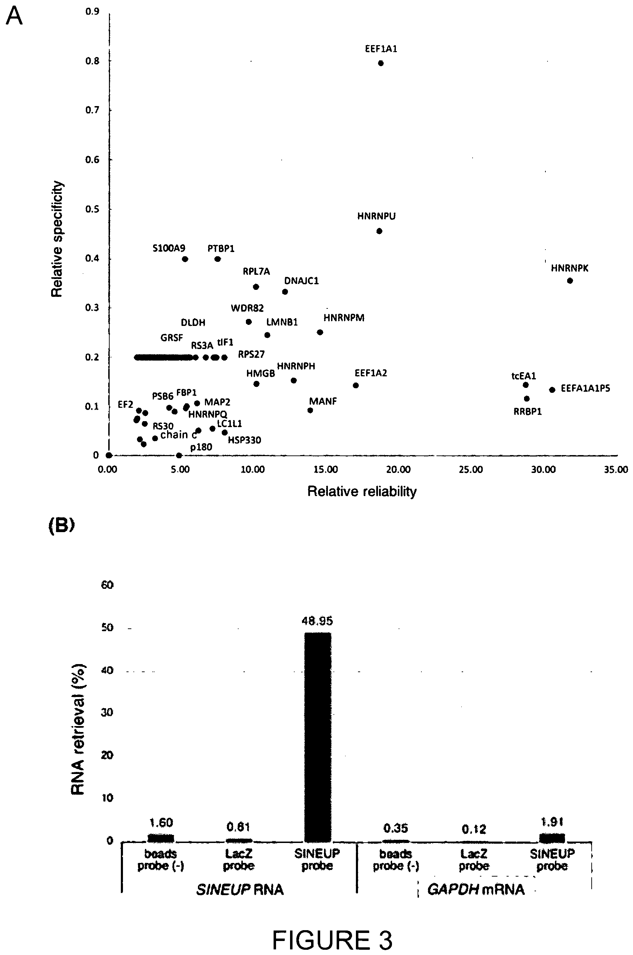

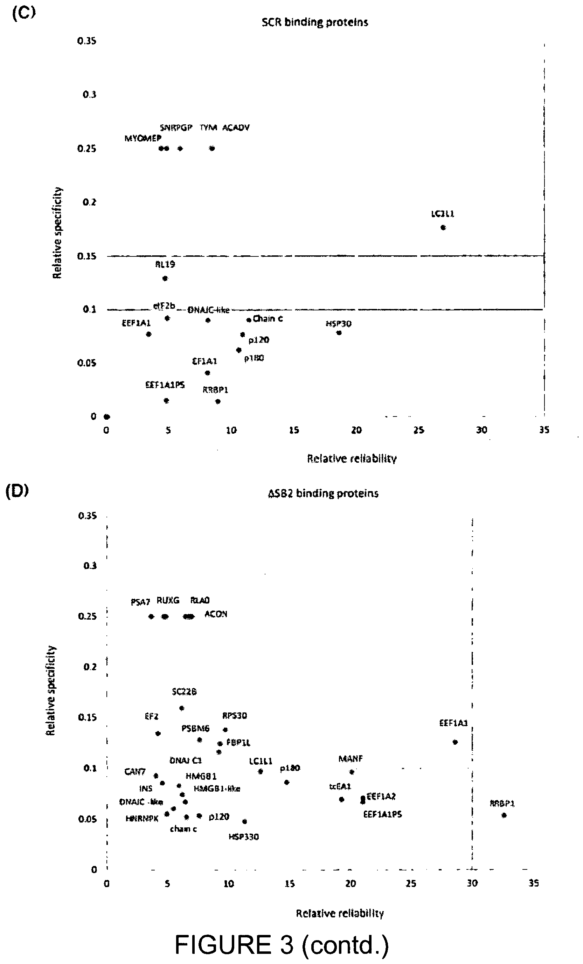

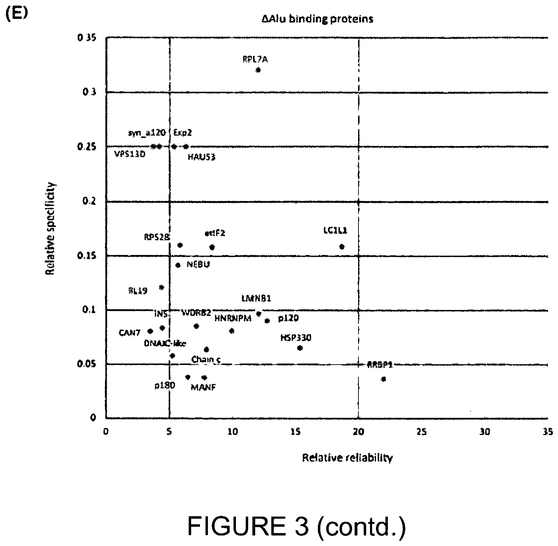

**FIGURE 3****: SINEUP-GFP RNA binding proteins.** A) Detected RNA binding proteins (RBPs) were plotted with a reliability of RBPs on the x axis that was calculated by the average of the SINEUP-GFP MASCOT score; reliability = sum of the MASCOT score/ n, and with a specificity of RBPs in target samples on the y axis that was the sum of the MASCOT score divided by the total MASCOT score of other mutants; specificity = (sum of the MASCOT score/sum of total other mutants MASCOT score)/n. n ≥ 3. Proteins that were detected by beads and LacZ probes were omitted. The proteins selected knocked down for the following siRNA mediated experiments are indicated in red. B) Percentage retrieval of RNA by modified ChIRP with SINEUP RNA probe. Biotinylated SINEUP-GFP probe specifically enriched. SINEUP-GFP RNA compared with the Magna ChIRP negative control probe (LacZ) and magnetic beads alone (probe (-)). GAPDH mRNA was tested as a negative control to assess non-specific interaction. (C-E) SINEUP-SCR (C), SINEUP-ΔSB2 (D), and SINEUP-ΔAlu (E) RBPs plotted according to relative reliability and specificity.

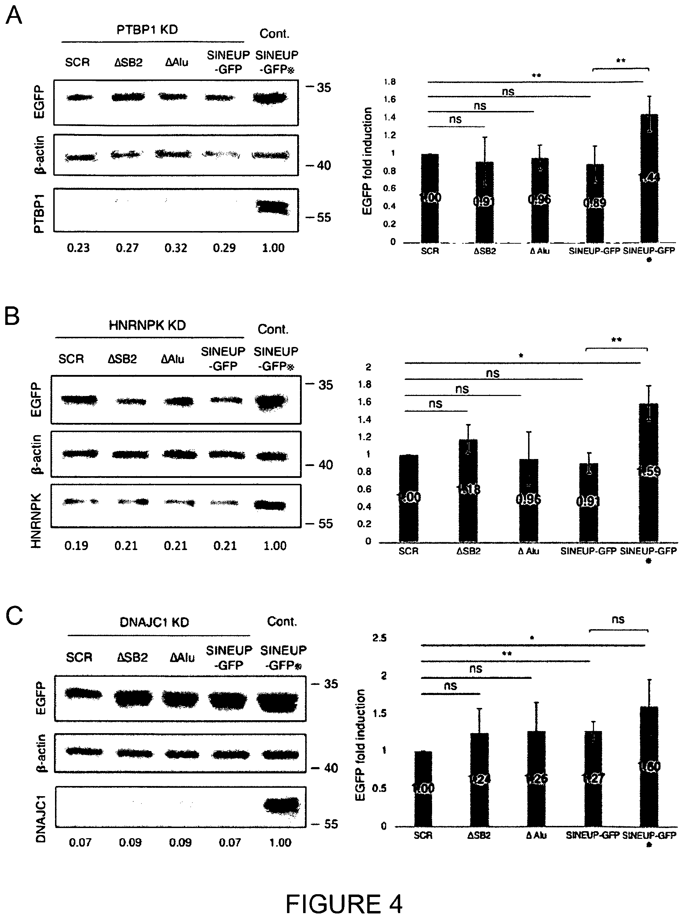

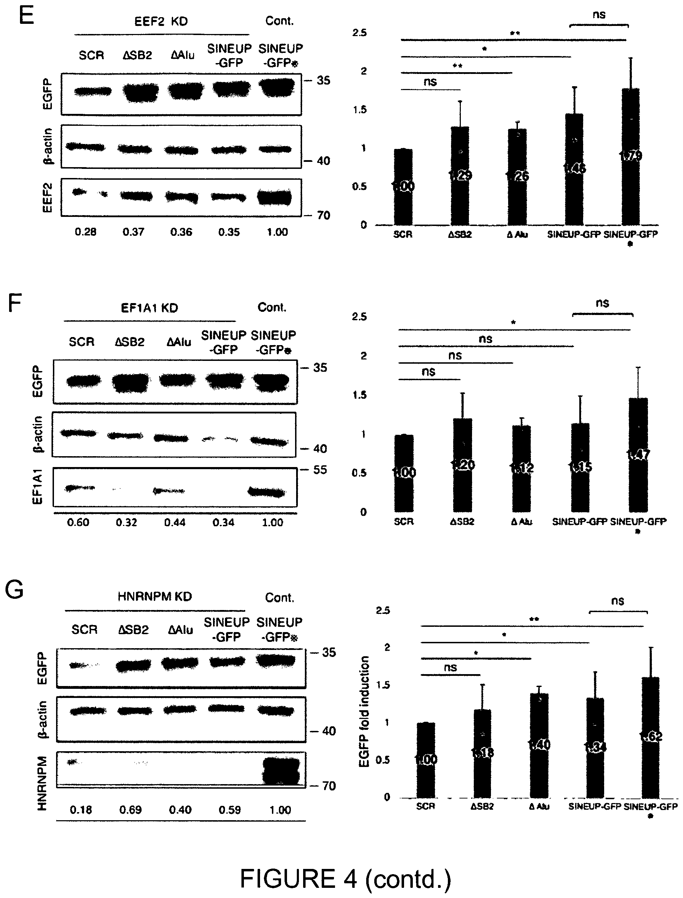

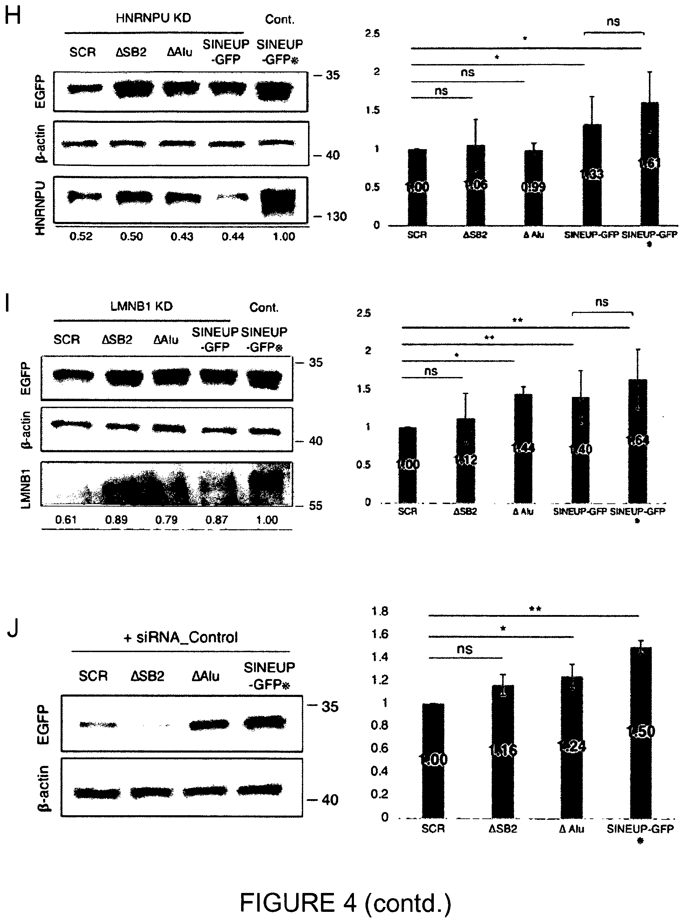

**FIGURE 4****: Knockdown of SINEUP RBPs.** (A-I) Representative Western blotting images of knockdown (KD) of different RNA binding proteins mediated by siRNA. Numbers under the bottom row indicate knockdown efficiency compared with cells co-transfected with SINEUP-GFP and negative control siRNA (SINEUP-GFP 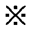 ). Protein levels of EGFP were quantified by Western blotting analysis. EGFP expression levels were normalized to that of β-actin. Fold induction of EGFP was calculated relative to cells transfected with the siRNA indicated in the panels. (J) Representative Western blotting images of transfection with negative control siRNA. **p < 0.01, *p < 0.05; ns: not significant by Student's t-test. Data are means ± SD of at least 3 independent experiments.

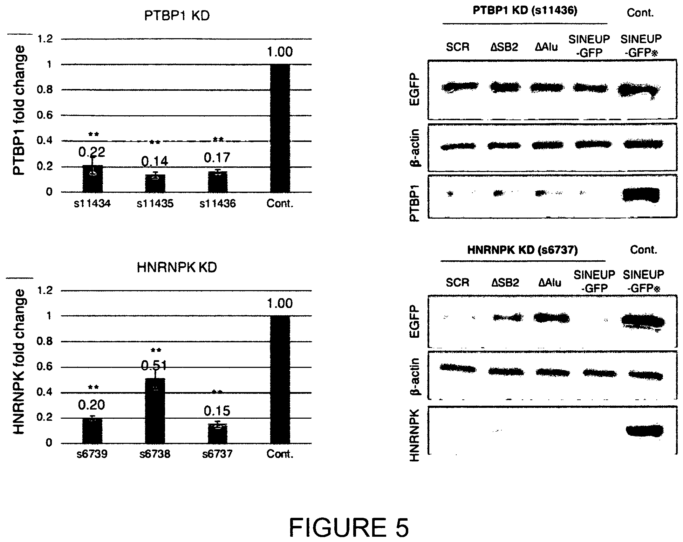

**FIGURE 5****: Knockdown by siRNAs targeting different regions of the RBPs.** Knockdown efficiency by several siRNAs of PTBP1 and HNRNPK compared with a SINEUP-GFP transfected control siRNA (SINEUP-GFP 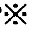 ). **p < 0.01 by Student's t-test. Data are means ± SD from at least 3 independent experiments. Graphs accompanied by representative Western blotting images showing knockdowns of domain mutant SINEUPs by a PTBP1 (s11436), and HNRNPK (s6737) siRNA.

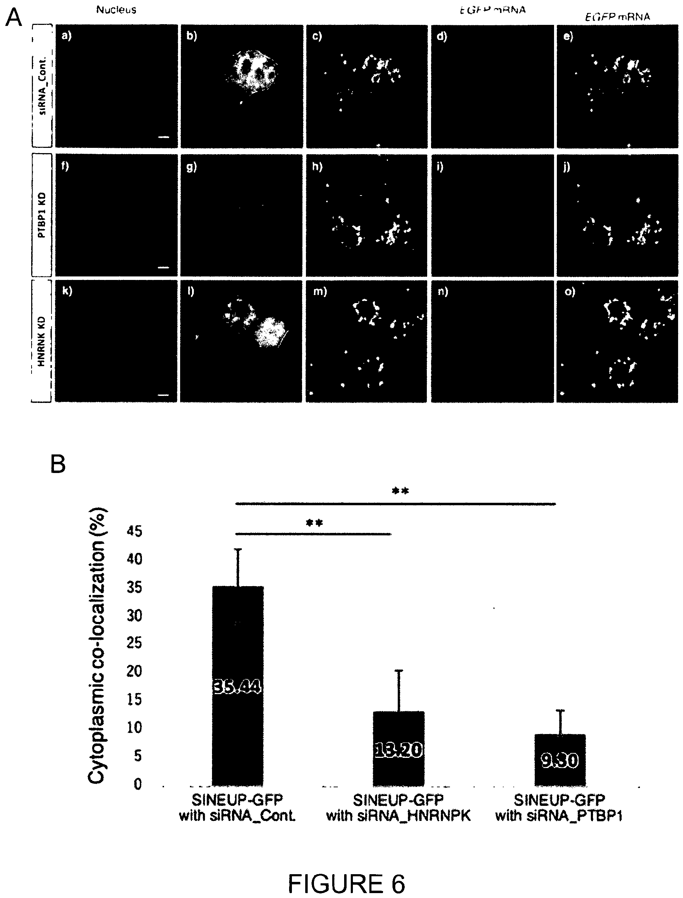

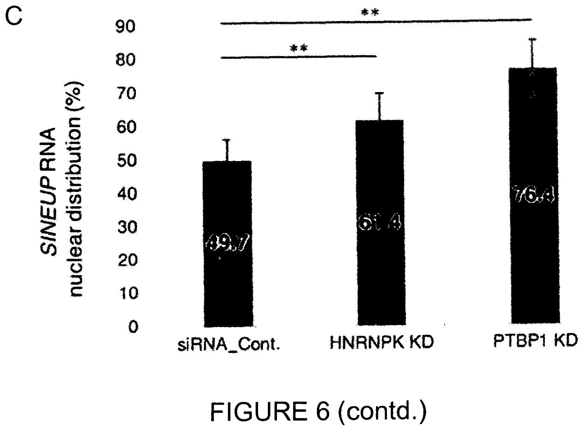

**FIGURE 6****:** (A) Representative FISH images following knockdown (KD) of PTBP1 (f-j) or HNRNPK (k-o) by siRNAs, and negative control siRNA; siRNA_Cont. (a-e). Bars indicate 5 µm. (B) Quantitative comparison of co-localization of EGFP mRNAs and SINEUP RNAs in the cytoplasm when PTBP1 (A, j) or HNRNPK (A, o) were knocked down. **p < 0.01 by Student's t-test. Data are means ± SD of 10 individual cell images. (C) Quantitative nuclear distribution of SINEUP-GFP RNAs following knockdown of PTBP1 (A, h) or HNRNPK (A, m) by siRNAs; the results are compared with the cells transfected negative control siRNA; siRNA_Cont (A, c). For both PTBP1 and HNRNPK, the ratio of SINEUP-GFP RNA levels in the nucleus and the cytoplasm were compared between the knockdowns and the negative control. **p < 0.01 by Student's t-test. Data are means ± SD of at least 10 independent cell images.

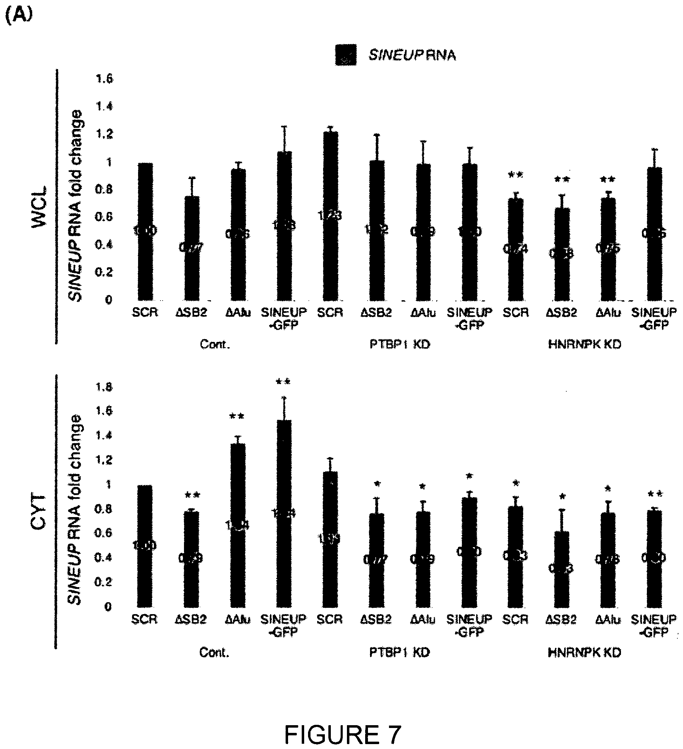

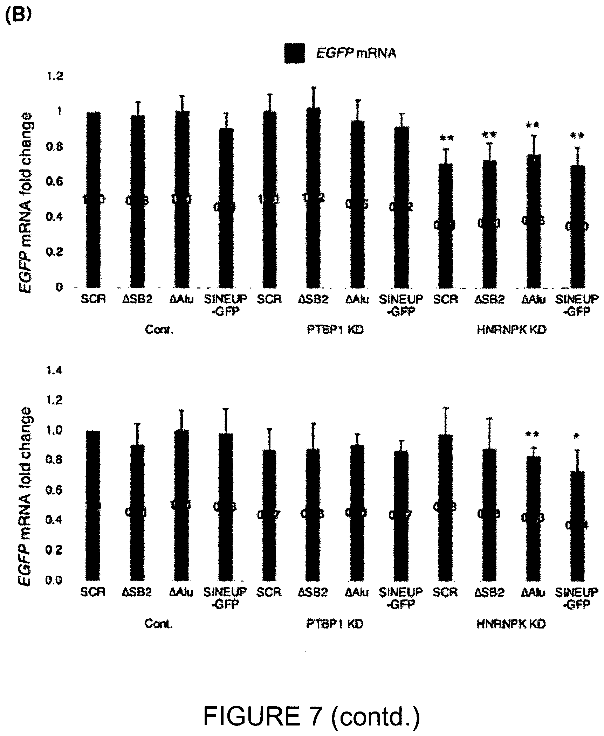

**FIGURE 7****: Quantification of RNA levels when PTBP1 or HNRNPK were knocked down.** Quantitative comparison of SINEUP RNA (A) and EGFP mRNA (B) expression levels between cells after knockdown (KD) of PTBP1 or HNRNPK, and non-knockdown of these proteins (Cont.). *p < 0.05, **p < 0.01 by Student's t-test. Data are means ± SD from at least 3 independent experiments. WCL indicates total RNAs from the whole cell lysate, and CYT indicates cytoplasmic RNAs from the cytoplasmic fraction.

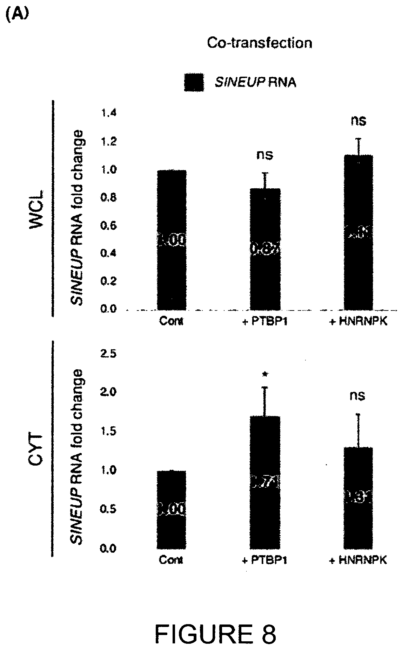

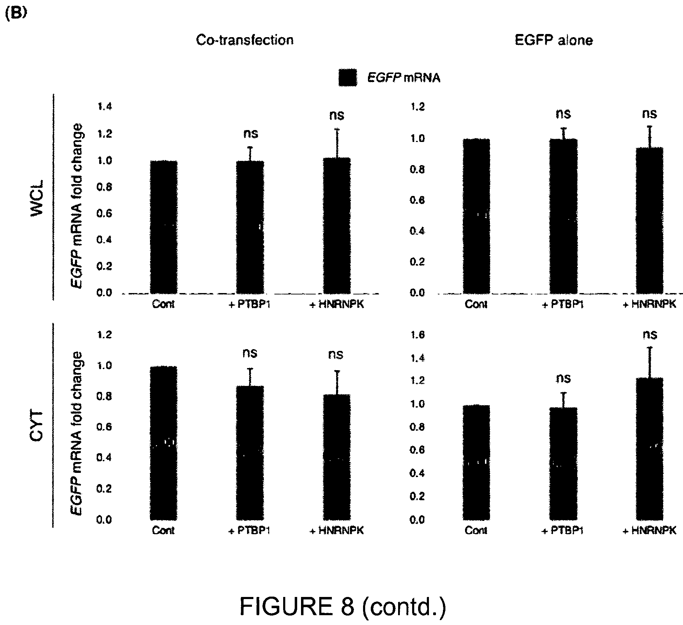

**FIGURE 8****: Quantification of RNA levels when PTBP1 or HNRNPK were overexpressed.** Quantitative comparison of SINEUP RNA (A) and EGFP mRNA (B) expression levels between cells overexpressing PTBP1 or HNRNPK, and non-overexpression of these proteins (Cont.). *p < 0.05, ns: not significant by Student's t-test. Data are means ± SD from at least 3 independent experiments. WCL indicates total RNAs from the whole cell lysate, and CYT indicates cytoplasmic RNAs from the cytoplasmic fraction.

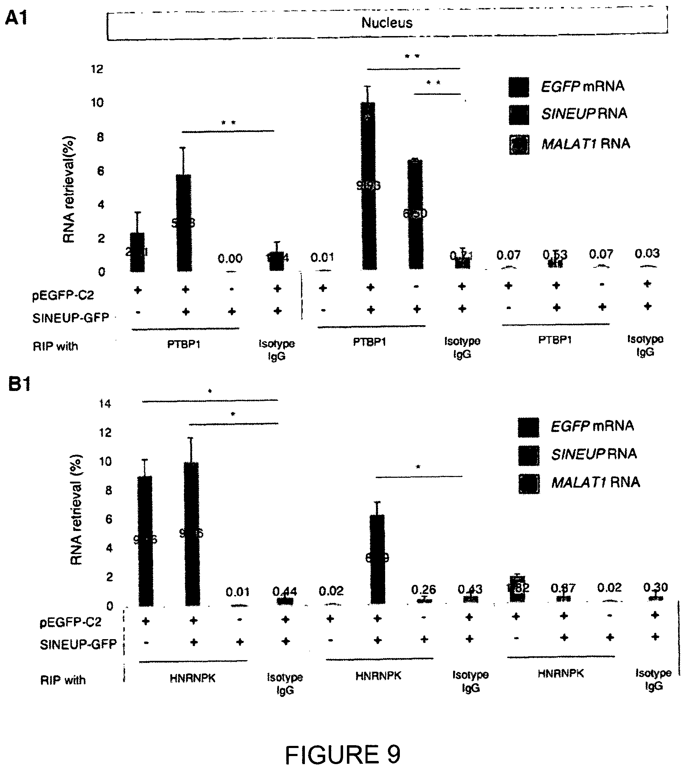

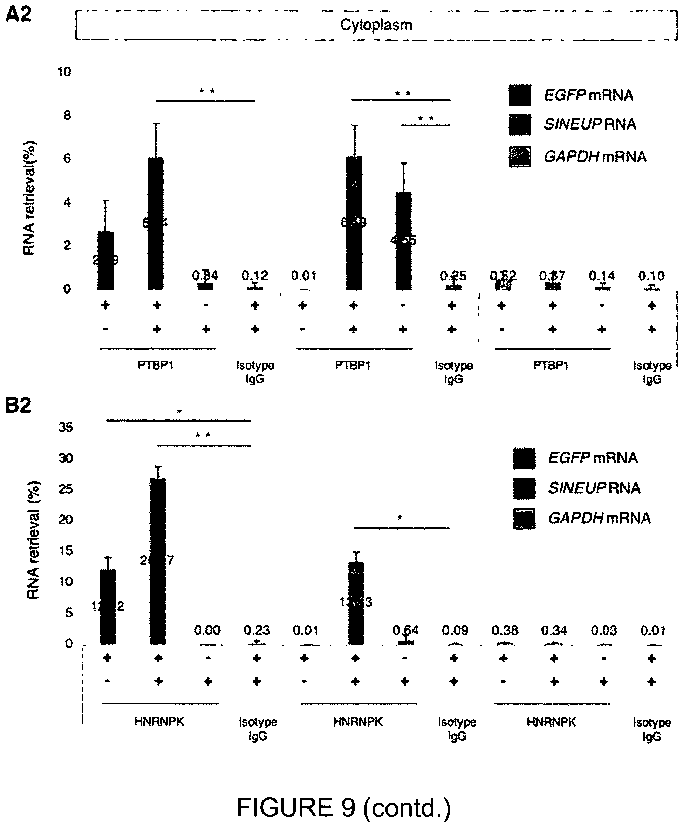

**FIGURE 9****: RNA immunoprecipitation.** RNA immunoprecipitation (RIP) with PTBP1 antibody in the nucleus (A1) and cytoplasm (A2). Isotype IgG was used as the negative antibody control. Transcripts from two of the most highly expressed housekeeping genes, MALAT1 and GAPDH, were used as negative controls in the nucleus and cytoplasm, respectively. *p < 0.05, **p < 0.01 by Student's t-test. Data are means ± SD of at least 3 independent experiments. RNA immunoprecipitation (RIP) with HNRNPK antibody or isotype immunoglobulin G (Isotype IgG; negative control) in the nucleus (B1) and cytoplasm (B2). Cell lysates co-transfected with pEGFP-C2 and SINEUP-GFP vectors or transfected with either vector alone were tested.

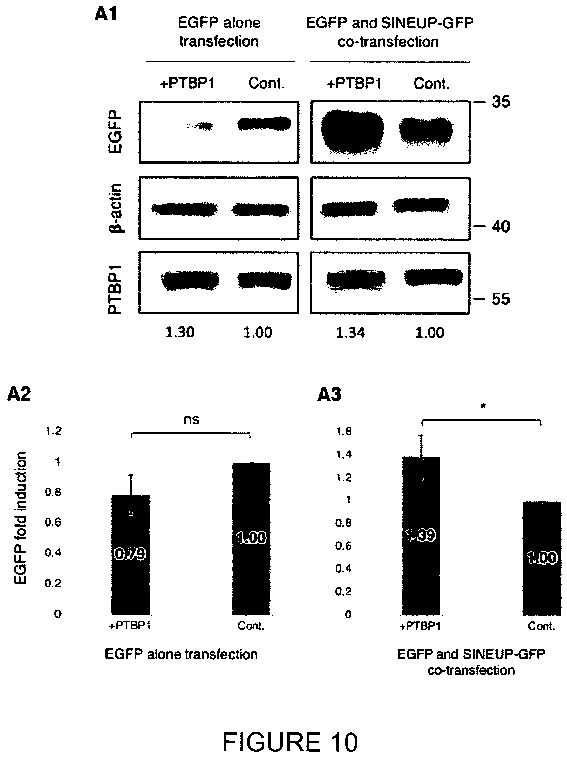

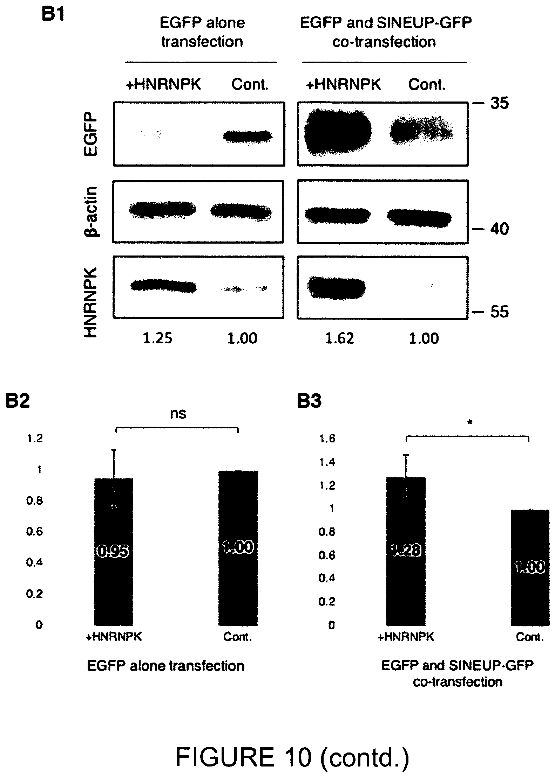

**FIGURE 10****: Overexpression of SINEUP RBPs.** Representative Western blotting images comparing EGFP expression after overexpression of PTBP1 (+PTBP1) (A1) or HNRNPK (+HNRNPK) (B1) with that in the non-overexpressing control (Cont.). Numbers under the image show the overexpression efficiency compared with controls. Graphs showing quantification of EGFP levels after non-/overexpression of PTBP1 (A2, A3) or HNRNPK (B2, B3) when cells were transfected with EGFP vector alone (A2, B2) or co-transfected with EGFP and SINEUP-GFP vectors (A3, B3). *p < 0.05, ns: not significant by Student's t-test. Data are means ± SD of at least 3 independent experiments.

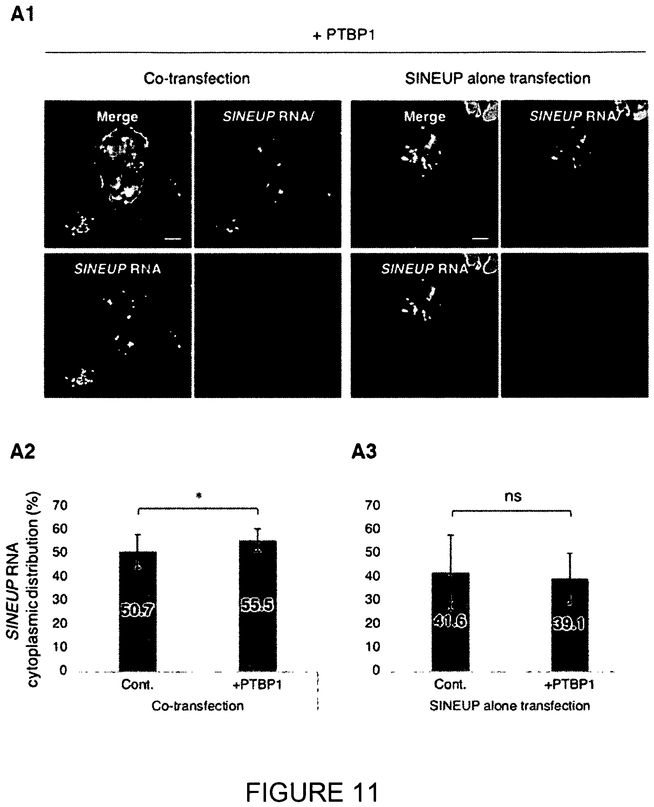

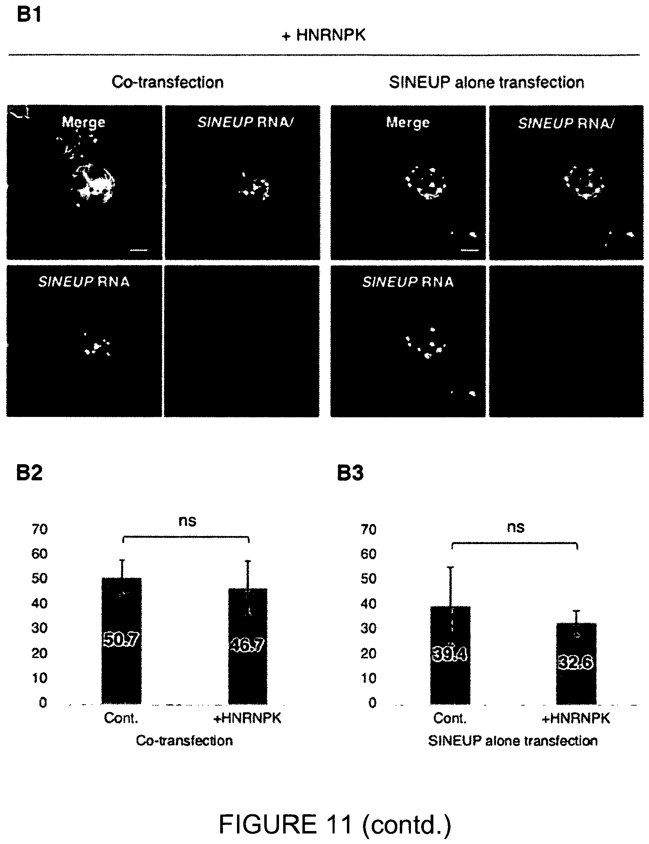

**FIGURE 11****: Subcellular distribution of SINEUP RNAs after overexpression of SINEUP RBPs.** Representative RNA FISH with immunofluorescence images of the subcellular distribution of SINEUP RNAs and SINEUP RBPs in cells overexpressing PTBP1 (+PTBP1) (A1) or HNRNPK (+HNRNPK) (B1). Images for cells co-transfected with EGFP and SINEUP-GFP vectors (left images, A1 and B1) were compared with cells transfected with SINEUP-GFP vector alone (right images, A1 and B1). Bars indicate 5 µm. Graphs showing quantitative comparison of SINEUP-GFP RNA distribution between cells overexpressing PTBP1 (A2, A3) or HNRNPK (B2, B3) and cells without overexpression (Cont.). Results for cells co-transfected with EGFP and SINEUP-GFP vectors (A2 and B2) and those transfected with SINEUP-GFP vector alone (A3, B3) are shown. SINEUP RNA signals were detected using Icy Spot Detector. The ratio of spots in the nucleus and the cytoplasm were compared between overexpression and non-overexpression of SINEUP RBPs. *p < 0.05 by Student's t-test. Data are means ± SD of at least 10 independent cell images.

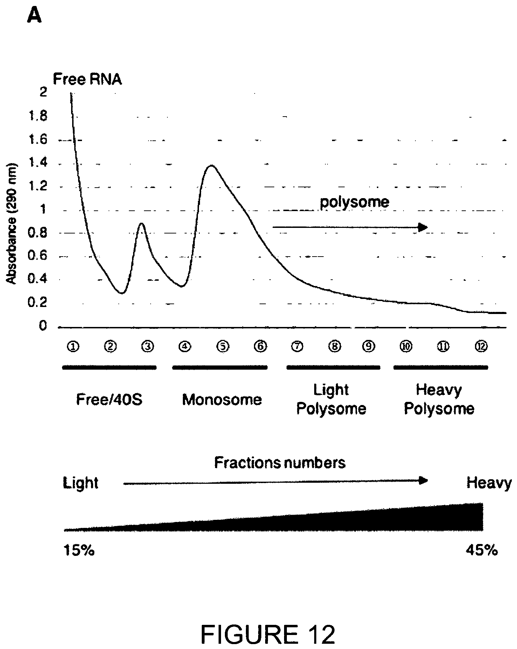

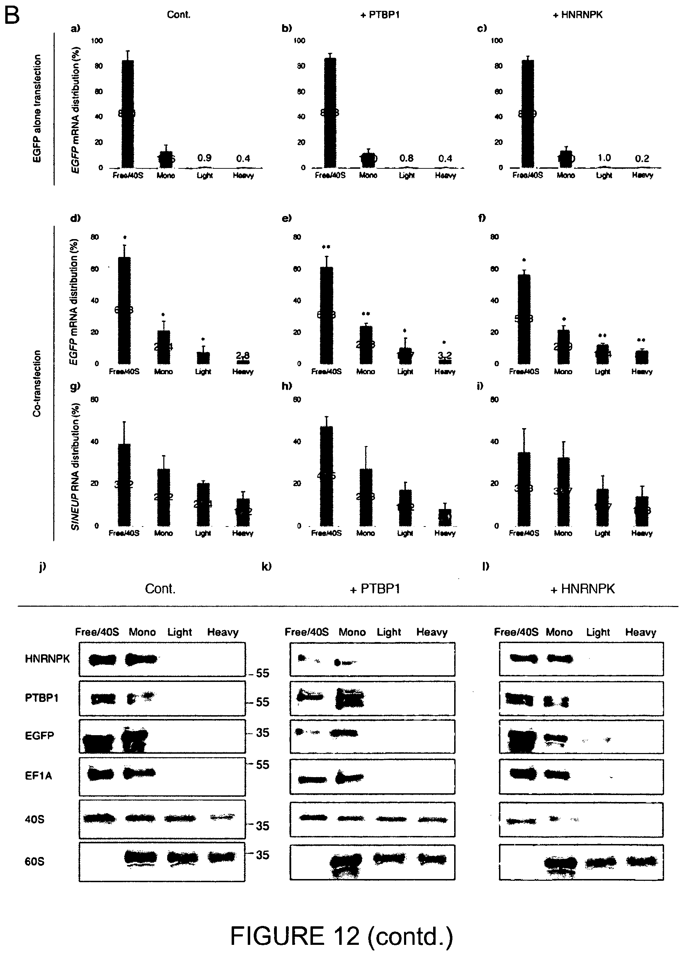

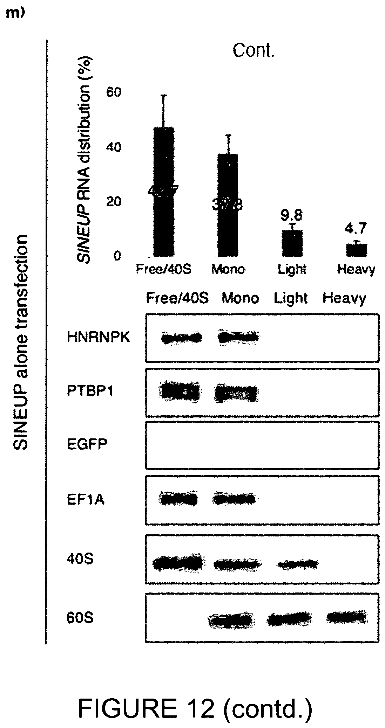

**FIGURE 12****: RNA distribution in polysome fractions obtained from cells overexpressing SINEUP RBPs.** A) Representative polysome gradient profile with optical density (290 nm). Fractions ① and ⑫ correspond to the 15% and 45% sucrose fractions, respectively. B) Polysome profiling of EGFP mRNA (a-f) and SINEUP-GFP RNA (g-i). RNA distribution was quantified by RT-qPCR. Each fraction of EGFP mRNA in cells co-transfected with EGFP and SINEUP-GFP vectors (d-f) was compared with the corresponding fraction in cells transfected with EGFP vector alone (a-c). *p < 0.05, **p < 0.01 by Student's t-test. Data are means ± SD from 3 independent experiments. Fractions of SINEUP RNA in cells co-transfected with EGFP and SINEUP-GFP vectors are shown in g-I, and those in cells transfected with SINEUP RNA alone are shown in m. The protein distribution in polysome fractions with non-/overexpressed PTBP1 or HNRNPK were tested (j-m). Equal volumes of solution were pooled as Free/40S (Free or 40S binding RNA fraction from ①-③ in A), Mono (monosome fraction from ④-⑥), Light (light polysome fraction from ⑦-⑨), and Heavy (Heavy polysome fraction from ⑩-⑫) and were applied to 10% SDS gels to detect each target protein. Western blotting images representative of least 3 independent experiments are shown.

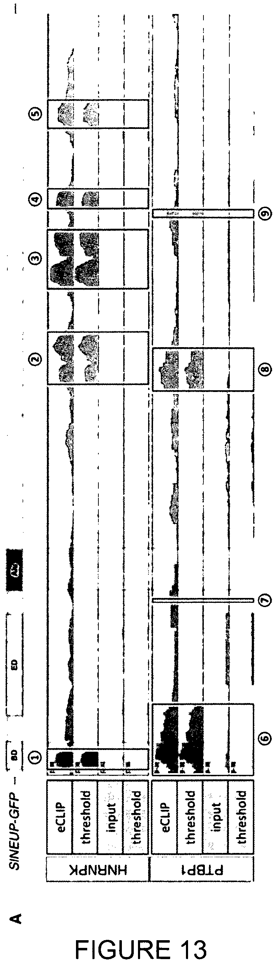

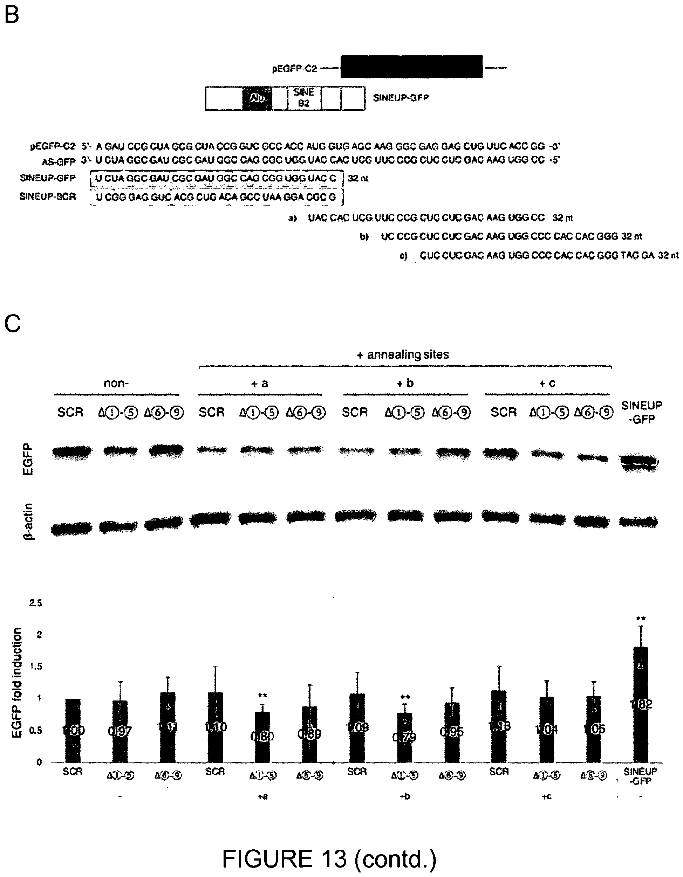

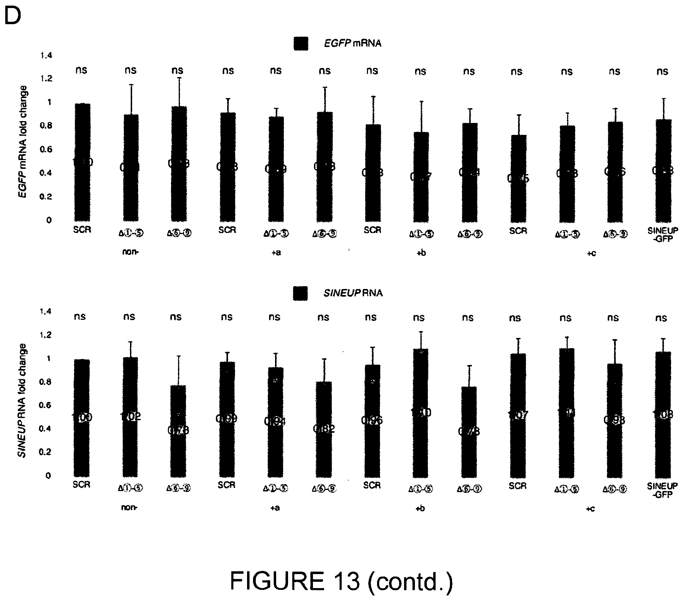

**FIGURE 13****: Identification of the SINEUPP RBPs binding regions by seCLIP-seq analysis.** A) Read coverage along SINEUP-GFP shown by seCLIP with HNRNPK or PTBP1. Labeled boxes show the identified binding regions with HNRNPK (①-⑤) and PTBP1 (⑥-⑨) on SINEUP-GFP transcripts. B) Schematic representation of annealing sites a), b) and c) with the SINEUP-GFP and SCR mutant are shown. C) Representative Western blotting image on the EGFP level (top) and quantification of the EGFP level (bottom). EGFP vector and the mutants were co-transfected in HEK-293T/17. **p < 0.01, ns: not significant, by Student's t-test. Data are means ± SD from at least 3 independent experiments. The SINEUP deletion mutants Δ①-⑤ (deleted HNRNPK binding regions from SINEUP-GFP), and Δ⑥-⑨ (deleted PTBP1 binding regions from SINEUP-GFP) are shown in A, and annealing sites are shown in B. D) Quantification of EGFP mRNA and SINEUP RNA levels following co-transfection with EGFP and SINEUP expression vectors. Data are means ± SD from at least 3 independent experiments. The SINEUP deletion mutants Δ①-⑤ (deleted HNRNPK binding regions from SINEUP-GFP), and Δ⑥-⑨ (deleted PTBP1 binding regions from SINEUP-GFP) are shown in A, and annealing sites were shown in B. ns: not significant by Student's t-test. Data are means ± SD from at least 3 independent experiments.

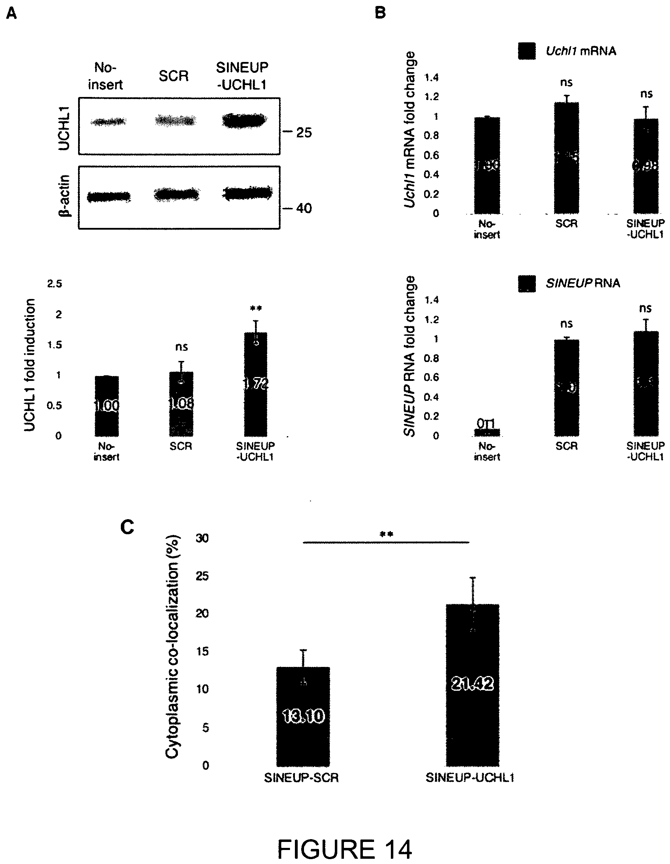

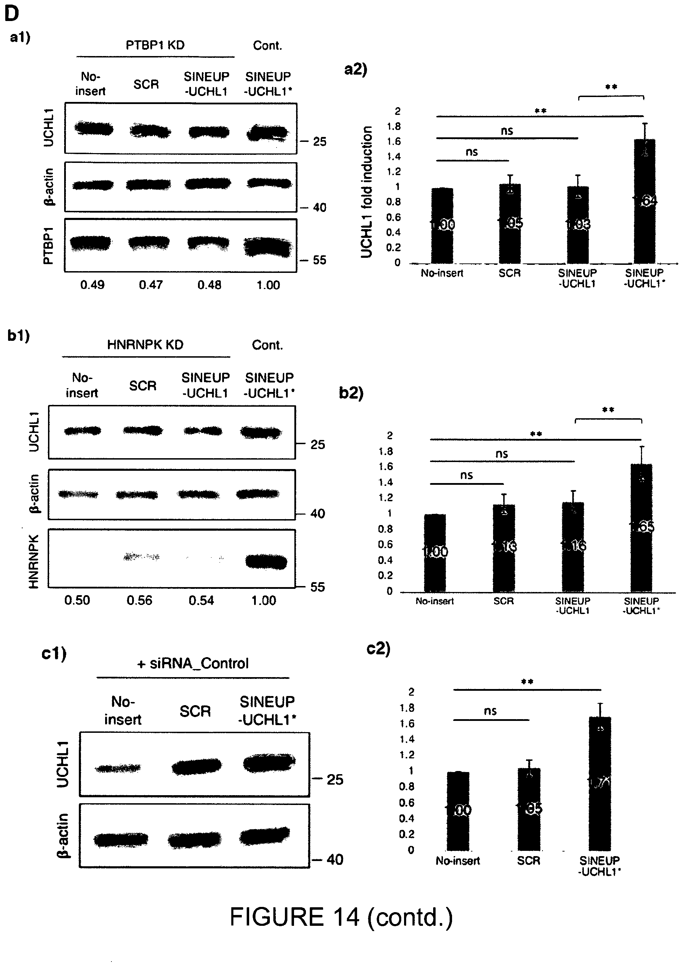

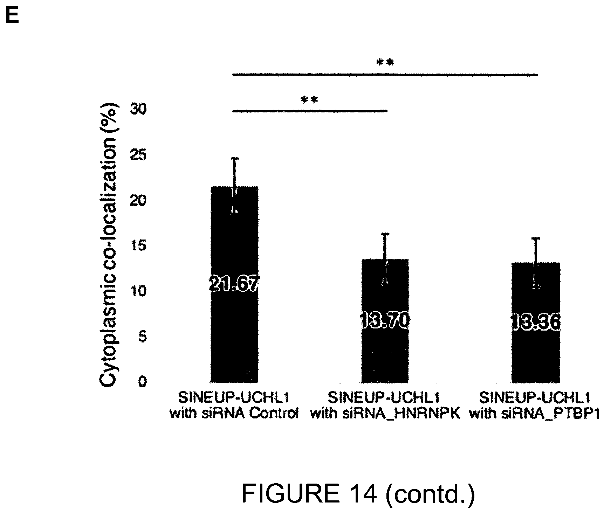

**FIGURE 14****: Enhancement of UCHL1 by synthetic SINEUP-UCHL1.** A) Translational up-regulation of UCHL1 by transfection of SINEUP-UCHL1 expression vectors. Representative Western blotting image on the UCHL1 protein level (top) and quantification of the UCHL1 level (bottom). **p < 0.01, ns: not significant, by Student's t-test. Data are means ± SD from at least 3 independent experiments. B) Quantification of the UCHL1 mRNA and SINEUP RNA levels following transfection with SINEUP expression vectors. Data are means ± SD from at least 3 independent experiments. C) Quantitative comparison of co-localization of Uchl1 mRNAs and SINEUP RNAs in the cytoplasm. **p < 0.01 by Student's t-test. Data are means ± SD of more than 10 individual cell images. D) Translational up-regulation of UCHL1 by transfection with SINEUP-UCHL1 expression vectors. Representative Western blotting images of knockdown (KD) of PTBP1 and HNRNPK mediated by siRNA_PTBP1 (a1,2) and siRNA HNRNPK (b1,2), respectively. Numbers under the bottom row indicate knockdown efficiency compared with the cells transfected with SINEUP-UCHL1 and negative control siRNA (c1,2, SINEUP-UCHL1*). E) Quantitative comparison of co-localization of Uchl1 mRNAs and SINEUP RNAs in the cytoplasm when PTBP1 or HNRNPK were knocked down. **p < 0.01 by Student's t-test. Data are means ± SD of more than 10 individual cell images.

### DETAILED DESCRIPTION

It is an object of the present invention to provide functional nucleic acid molecules with enhanced protein translation activity. The results presented herein indicate that intermolecular interactions between SINEUP RNAs and the RBPs contribute to the translational up-regulation of the target mRNA. Therefore, the invention incorporates RBP binding sites into SINEUP molecules in order to improve protein translation activity. The functional nucleic acid molecules may therefore be targeted to an mRNA sequence to enhance protein translation.

### Definitions

The "functional nucleic acid molecule" referred to herein is a synthetic molecule described by the invention. In particular, "functional nucleic acid molecule" describes a nucleic acid molecule (e.g. DNA or RNA) that is capable of enhancing translation of a target mRNA of interest. The term "functional RNA molecule" refers to wherein the functional nucleic acid molecule is formed of RNA and the RNA molecule is capable of enhancing the translation of a target mRNA of interest. The functional molecules described herein may be referred to as trans-acting molecules.

References to "RNA binding protein" or "RBP" refers to proteins that bind RNA through one or more globular RNA binding protein domains (RBDs, also referred to as RNA binding protein regions) and change the fate or function of the bound RNAs. The term includes global RBPs (those that bind to nearly all mRNAs without distinguishing unique sequences), group-specific RBPs (those that associate with subsets of the global mRNA population), and type-specific RBPs (those that recognize a highly unique mRNA sequence, in some cases present in only one mRNA, with high specificity). The RBP may bind single stranded or double stranded RNA. See for example, the database of RNA binding protein specificities (RBPDB) as described in Cook et al. (2011) Nucleic Acid Research 39: D301-D308, provides examples of known RBPs and is herein incorporated by reference.

The term "SINE" (Short Interspersed Nuclear Element) refers to an interspersed repetitive sequence (a) which encodes a protein having neither reverse-transcription activity nor endonuclease activity or the like and (b) whose complete or incomplete copy sequences exist abundantly in genomes of living organisms.

The term "SINE B2 element" is defined in WO 2012/133947, where specific examples are also provided (see table starting on page 69 of the PCT publication). The term is intended to encompass both SINE B2 elements in direct orientation and in inverted orientation relative to the 5' to 3' orientation of the functional nucleic acid molecule. SINE B2 elements may be identified, for example, using programs like RepeatMask as published (Bedell et al. Bioinformatics. 2000 Nov; 16(11): 1040-1. MaskerAid: a performance enhancement to RepeatMasker). A sequence may be recognizable as a SINE B2 element by returning a hit in a Repbase database with respect to a consensus sequence of a SINE B2, with a Smith-Waterman (SW) score of over 225, which is the default cutoff in the RepeatMasker program. Generally a SINE B2 element is not less than 20 bp and not more than 400 bp. Preferably, the SINE B2 is derived from tRNA.

By the term "functionally active fragment of a SINE B2 element" there is intended a portion of sequence of a SINE B2 element that retains protein translation enhancing efficiency. This term also includes sequences which are mutated in one or more nucleotides with respect to the wild-type sequences, but retain protein translation enhancing efficiency. The term is intended to encompass both SINE B2 elements in direct orientation and in inverted orientation relative to the 5' to 3' orientation of the functional nucleic acid molecule.

The terms "internal ribosome entry site (IRES) sequence" and "internal ribosome entry site (IRES) derived sequence" are defined in WO 2019/058304. IRES sequences recruit the 40S ribosomal subunit and promote cap-independent translation of a subset of protein coding mRNAs. IRES sequences are generally found in the 5' untranslated region of cellular mRNAs coding for stress-response genes, thus stimulating their translation in *cis.* It will be understood by the term "IRES derived sequence" there is intended a sequence of nucleic acid with a homology to an IRES sequence so as to retain the functional activity thereof, i.e. a translation enhancing activity. In particular, the IRES derived sequence can be obtained from a naturally occurring IRES sequence by genetic engineering or chemical modification, e.g. by isolating a specific sequence of the IRES sequence which remains functional, or mutating/deleting/introducing one or more nucleotides in the IRES sequence, or replacing one or more nucleotides in the IRES sequence with structurally modified nucleotides or analogs. More in particular, the skilled in the art would know that an IRES derived sequence is a nucleotide sequence capable of promoting translation of a second cistron in a bicistronic construct. Typically, a dual luciferase (Firefly luciferase, Renilla Luciferase) encoding plasmid is used for experimental tests. A major database exists, namely IRESite, for the annotation of nucleotide sequences that have been experimentally validated as IRES, using dual reporter or bicistronic assays (http://iresite.org/IRESite_web.php). Within the IRESite, a web-based tool is available to search for sequence-based and structure-based similarities between a query sequence of interest and the entirety of annotated and experimentally validated IRES sequences within the database. The output of the program is a probability score for any nucleotide sequence to be able to act as IRES in a validation experiment with bicistronic constructs. Additional sequence-based and structure-based web-based browsing tools are available to suggest, with a numerical predicting value, the IRES activity potentials of any given nucleotide sequence (http://rna.informatik.uni-freiburg.de/; http://regrna.mbc.nctu.edu.tw/index1.php).

By the term "miniSINEUP" there is intended a nucleic acid molecule comprising (or consisting of) a binding domain (i.e. a complementary sequence to target mRNA), optionally a spacer sequence, and any SINE or SINE-derived sequence or IRES or IRES-derived sequence as the effector domain (Zucchelli et al., Front Cell Neurosci., 9: 174, 2015).

By the term "microSINEUP" there is intended a nucleic acid molecule comprising (or consisting of) a binding domain (i.e. a complementary sequence to target mRNA), optionally a spacer sequence, and a functionally active fragment of the SINE or SINE-derived sequence or IRES-derived sequence. For example, the functionally active fragment may be a 77 bp sequence corresponding to nucleotides 44 to 120 of the SINE B2 element in AS Uchl1.

Polypeptide or polynucleotide sequences are said to be the same as or "identical" to other polypeptide or polynucleotide sequences, if they share 100% sequence identity over their entire length. Residues in sequences are numbered from left to right, i.e. from N- to C- terminus for polypeptides; from 5' to 3' terminus for polynucleotides.

For the purposes of comparing two closely-related polynucleotide sequences, the "% sequence identity" between a first nucleotide sequence and a second nucleotide sequence may be calculated using NCBI BLAST, using standard settings for nucleotide sequences (BLASTN). For the purposes of comparing two closely-related polypeptide sequences, the "% sequence identity" between a first polypeptide sequence and a second polypeptide sequence may be calculated using NCBI BLAST, using standard settings for polypeptide sequences (BLASTP). A "difference" between sequences refers to an insertion, deletion or substitution of a single nucleotide in a position of the second sequence, compared to the first sequence. Two sequences can contain one, two or more such differences. Insertions, deletions or substitutions in a second sequence which is otherwise identical (100% sequence identity) to a first sequence result in reduced % sequence identity.

### Functional Molecules

According to a first aspect of the invention, there is provided a functional nucleic acid molecule comprising:
(a) at least one target determinant sequence comprising a sequence reverse complementary to a target mRNA sequence for which protein translation is to be enhanced;
(b) at least one sequence encoding an RNA binding protein region; and
(c) at least one regulatory sequence comprising a SINE B2 element or a functionally active fragment of a SINE B2 element or an internal ribosome entry site (IRES) sequence or an IRES derived sequence.

RNA binding proteins are known in the art and are identified, for example in the RNA-Binding Protein DataBase (RBPDB). RBPs typically contain RNA binding domains (RBDs), such as the RNA Recognition Motif (RRM) and the K homology (KH) domain. The RBP sequence may be derived from various sources of RBPs, such as human, mouse, fly (*Drosophila melanogaster*) and worm (*Caenorhabditis elegans*), in particular human RBPs.

The RNA binding protein region may also be referred to as an RNA binding protein motif. In one embodiment, the RNA binding protein region comprises a sequence recognised by a RNA binding domain selected from: RNA Recognition Motif, CCCH zinc finger, K Homology, Like-Sm domain, C2H2 zinc finger, Ribosomal protein S1-like, Cold-shock domain, Lupus La RNA-binding domain, Pumilio-like repeat, Pseudouridine synthase and archaeosine transglycosylase (PUA domain), Surp module/SWAP, Sterile Alpha Motif, YTH domain, PWI domain, THUMP domain and TROVE module. In particular, the RNA binding protein region comprises a sequence recognised by a RNA binding domain selected from: RNA Recognition Motif, CCCH zinc finger or K Homology, in particular RNA Recognition Motif or K Homology.

RNA binding protein regions are well understood in the art and it has been found that many RBPs bind similar sequence motifs. Regions bound by RBPs have been identified, for example, by mCrossBase using ENCODE eCLIP data (see Feng et al. (2019) Mol. Cell 74: 1189-1204 and the online mCrossBase database) or determined by the method in published papers (see Dominguez et al. (2018) Mol. Cell 70: 854-867). Exemplary motifs that have been identified as RNA binding protein regions are included in Table 2. In one embodiment, RNA binding protein region comprises one or more sequences provided in Table 2 (or an RNA sequence encoded by said sequence).

In one embodiment, the RNA binding protein region comprises a sequence recognised by an RNA binding protein selected from: PTBP1, HNRNPK, DNAJC1, EEF2 , EF1A1, HNRNPM, HNRNPU and LMNB1. In preferred embodiments, the sequence is recognised by an RNA binding protein selected from: PTBP1 and HNRNPK. Therefore, the RNA binding protein region may be selected from a HNRNPK binding region and a PTBP1 binding region.

PTBP1 (polypyrimidine tract binding protein-1, also known as HNRNPI) is a multifunctional RNA binding protein that participates in alternative splicing, mRNA stabilization, and nucleocytoplasmic shuttling by binding to the polypyrimidine-rich tract in pre-mRNAs. PTBP1 is known as a binding factor for TOP mRNA, which contains a 5' terminal oligopyrimidine tract (5'TOP) mostly found in mRNAs encoding ribosomal protein and elongation factors, and regulates translation of the target TOP mRNA as a cis-acting regulator. LARP1, which is known as a 5'TOP mRNA binding protein, stabilizes the target 5'TOP mRNAs by forming complexes with the 40S ribosome subunit. The data described herein implies that a multimer made up of SINEUP RNA, PTBP1, 40S and the target mRNA may contribute to target mRNA stabilization.

HNRNPK (heterogeneous nuclear ribonucleoprotein K) has three KH (K homology) domains, which bind RNAs, and unique nuclear localization signals with bi-directional transport, that enables its export to the nuclear envelope with target mRNAs. HNRNPK regulates the target mRNA's translation positively or negatively, depending on the target mRNA. As an example of positive regulation, HNRNPK bound to VEGF mRNA and stimulated the ribosome to bind the mRNA resulting in a shift to heavier polysomes. In contrast, in a case of negative regulation, HNRNPK blocked monosome assembly by binding to the 3' UTR of c-Src mRNA thereby repressing the translation. In the data presented herein, HNRNPK shifted to heavier polysomes with EGFP mRNA when HNRNPK was overexpressed and up-regulated EGFP mRNA translation, supporting positive regulation. Furthermore, HNRNPK contributed to ribosome assembly only when EGFP mRNA and SINEUP-GFP RNA co-existed.

Both HNRNPK and PTBP1 are classed as heterogeneous nuclear ribonucleoproteins (RNPs), which mainly participate in alternative mRNA splicing, conformation of RNP assembly to compact transcripts in the nucleus, and nucleocytoplasmic shuttling.

The RNA binding protein region comprises a sequence which is sufficient in length to bind to an RNA binding protein. Therefore, the target binding sequence may be at least 4 nucleotides long, such as at least 5 nucleotides long, such as least 7 nucleotides long. Furthermore, the RNA binding protein region may be less than 150 nucleotides long, preferably less than 120 nucleotides long, less than 100 nucleotides long, less than 80 nucleotides long, less than 60 nucleotides long or less than 50 nucleotides long. In one embodiment, the RNA binding protein region is between 4 and 150 nucleotides in length, such as between 5 and 50 nucleotides long.

In one embodiment, the HNRNPK binding region is selected from a sequence with at least 60% sequence identity to SEQ ID NO: 1-5. In a further embodiment, the HNRNPK binding region is selected from a sequence with at least 75% sequence identity, such as at least 80%, 85%, 90%, 95%, 97%, 99% or 100% sequence identity to SEQ ID NO: 1-5.

In one embodiment, the PTBP1 binding region is selected from a sequence with at least 60% sequence identity to SEQ ID NO: 6-8. In a further embodiment, the PTBP1 binding region is selected from a sequence with at least 75% sequence identity, such as at least 80%, 85%, 90%, 95%, 97%, 99% or 100% sequence identity to SEQ ID NO: 6-8.

In one embodiment, the RNA binding protein region is located at the 5 '-end, the 3 '-end, or both ends of said functional nucleic acid molecule. The RNA binding protein region may be located at the 5 '-end or the 3 '-end of the target determinant sequence. In particular, the RNA binding protein region may be located 5' of the target determinant sequence. The RNA binding protein region is separate from the other sequences included in the functional nucleic acid molecule (in particular, the RNA binding protein region is separate to the target determinant sequence), therefore it will be understood that in this embodiment the RNA binding protein region is present in the functional nucleic acid molecule in addition to the target determinant and regulatory sequences.

In one embodiment, the functional nucleic acid molecule further comprises at least one linker sequence between the target determinant sequence and the regulatory sequence. SEQ ID NO: 9 is a non-limiting example of the spacer/linker sequence.

In one embodiment, the functional nucleic acid molecule is circular. This conformation leads to a much more stable molecule that is degraded with greater difficulty within the cell (exonucleases cannot degrade circular molecules) and therefore remains active for a longer time.

In one embodiment, the functional nucleic acid molecule comprises a 3'-polyadenylation (polyA) tail. A "3'-polyA tail" refers to a long chain of adenine nucleotides added to the 3'-end of the transcription which provides stability to the RNA molecule and can promote translation.

In one embodiment the functional nucleic acid molecule comprises a 5'-cap. A "5'-cap" refers to an altered nucleotide at the 5'-end of the transcript which provides stability to the molecule, particularly from degradation from exonucleases, and can promote translation. Most commonly, the 5'-cap is a 7-methylguanylate cap (m7G), *i.e.* a guanine nucleotide connected to the RNA via a 5' to 5' triphosphate linkage and methylated on the 7 position.

### Regulatory sequence

The regulatory sequence has protein translation enhancing efficiency. The increase of the protein translation efficiency indicates that the efficiency is increased as compared to a case where the functional nucleic acid molecule according to the present invention is not present in a system. In one embodiment, expression of the protein encoded by the target mRNA is increased by at least 1.2 fold, such as at least 1.5 fold, in particular at least 2 fold.

In one embodiment, the regulatory sequence is located 3' of the target binding sequence. The regulatory sequence may be in a direct or inverted orientation relative to the 5' to 3' orientation of the functional nucleic acid molecule. Reference to "direct" refers to the situation in which the regulatory sequence is embedded (inserted) with the same 5' to 3' orientation as the functional nucleic acid molecule. Instead, "inverted" refers to the situation in which the regulatory sequence is 3' to 5' oriented relative to the functional nucleic acid molecule.

In one embodiment, the regulatory sequence comprises a SINE B2 element or a functionally active fragment of a SINE B2 element. The SINE B2 element is preferably in an inverted orientation relative to the 5' to 3' orientation of the functional nucleic acid molecule, i.e. an inverted SINE B2 element. As mentioned in the definitions section, inverted SINE B2 elements are disclosed and exemplified in WO 2012/133947.

Short fragments of the regulatory sequence (such as a SINE B2 element) are particularly useful when providing functional RNA molecules for use as a nucleic acid therapeutic. RNA molecules are highly unstable in living organisms, therefore stability provided by the chemical modifications as described herein, is more effective for shorter RNA molecules. Therefore, in one embodiment, the regulatory sequence comprises a functionally active fragment which is less than 250 nucleotides, such as less than 100 nucleotides.

Preferably, the at least one regulatory sequence comprises a sequence with at least 90% sequence identity, preferably at least 95% sequence identity, more preferably 100% sequence identity with a sequence selected from the group consisting of SEQ ID NO: 10-60. In one embodiment, the at least one regulatory sequence consists of a sequence with at least 90% sequence identity, preferably at least 95% sequence identity, more preferably 100% sequence identity with a sequence selected from the group consisting of SEQ ID NO: 10-60.

SEQ ID NO: 10 (the 167 nucleotide variant of the inverted SINE B2 element in AS Uchl1) and SEQ ID NO: 11 (the 77 nucleotide variant of the inverted SINE B2 element in AS Uchl1 that includes nucleotides 44 to 120) are particularly preferred.

Other inverted SINE B2 elements and functionally active fragments of inverted SINE B2 elements are SEQ ID NO: 12-60. Experimental data showing the protein translation enhancing efficiency of these sequences is not explicitly shown in the present patent application, but is disclosed in a previous patent application in the name of the same applicant. SEQ ID NO: 12-60 can therefore also be used as regulatory sequences in molecules according to the present invention.

SEQ ID NO: 12-15, 17-20 and 27 are functionally active fragments of inverted SINE B2 transposable element derived from AS Uchl1. The use of functional fragments reduces the size of the regulatory sequence which is advantageous if used in an expression vector (e.g. viral vectors which may be size-limited) because this provides more space for the target sequence and/or expression elements.

SEQ ID NO: 16 is a full length 183 nucleotide (nt) inverted SINE B2 transposable element derived from AS Uchl1. SEQ ID NO: 21-26, 28, 29, 48-51 are mutated functionally active fragments of inverted SINE B2 transposable element derived from AS Uchl1.

SEQ ID NO: 30-34, 37-47 are different SINE B2 transposable elements. SEQ ID NO: 35 and 36 are sequences in which multiple inverted SINE B2 transposable element have been inserted.

Alternatively, the regulatory sequence comprises an IRES sequence or an IRES derived sequence. Therefore, in one embodiment, the regulatory sequence comprises an IRES sequence or an IRES derived sequence. Said sequence enhances translation of the target mRNA sequence.

Several IRESs having sequences ranging from 48 to 576 nucleotides have been tested with success, e.g. human Hepatitis C Virus (HCV) IRESs (e.g. SEQ ID NO: 61 and 62), human poliovirus IRESs (e.g. SEQ ID NO: 63 and 64), human encephalomyocarditis (EMCV) virus (e.g. SEQ ID NO: 65 and 66), human cricket paralysis (CrPV) virus (e.g. SEQ ID NO: 67 and 68), human Apaf-1 (e.g. SEQ ID NO: 69 and 70), human ELG-1 (e.g. SEQ ID NO: 71 and 72), human c-MYC (e.g. SEQ ID NO: 73-76), human dystrophin (DMD) (e.g. SEQ ID NO: 77 and 78).

Such sequences have been disclosed, defined and exemplified in WO 2019/058304. Preferably, such sequences have at least 90% sequence identity, preferably at least 95% sequence identity, more preferably 100% sequence identity to any of SEQ ID NO: 61-78.

### Target determinant sequence

The target determinant sequence (also referred to as the target binding sequence) is the portion of the functional nucleic acid molecule that binds to the target mRNA.

In WO 2012/133947 it was already shown that the target binding sequence needs to have only about 60% similarity with a sequence reverse complementary to the target mRNA. As a matter of fact, the target binding sequence can even display a large number of mismatches and retain activity.

The target binding sequence comprises a sequence which is sufficient in length to bind to the target mRNA transcript. Therefore, the target binding sequence may be at least 10 nucleotides long, such as at least 14 nucleotides long, such as least 18 nucleotides long. Furthermore, the target binding sequence may be less than 250 nucleotides long, preferably less than 200 nucleotides long, less than 150 nucleotides long, less than 100 nucleotides long, less than 80 nucleotides long, less than 60 nucleotides long or less than 50 nucleotides long. In one embodiment, the target binding sequence is between 4 and 50 nucleotides in length, such as between 18 and 44 nucleotides long.

The target binding sequence may be designed to hybridise with the 5'-untranslated region (5' UTR) of the target mRNA sequence. In one embodiment, the sequence is reverse complementary to 0 to 50 nucleotides, such as 0 to 40, 0 to 30, 0 to 21 or 0 to 14 nucleotides of the 5' UTR. Alternatively, or in combination, the target binding sequence may be designed to hybridise to the coding sequence (CDS) of the target mRNA sequence. In one embodiment, the sequence is reverse complementary to 0 to 40 nucleotides, such as 0 to 32, 0 to 18 or 0 to 4 nucleotides of the CDS.

The target binding sequence may be designed to hybridise to a region upstream of an AUG site (start codon), such as a start codon within the CDS, of the target mRNA sequence. In one embodiment, the sequence is reverse complementary to 0 to 80 nucleotides, such as 0 to 70 or 0 to 40 nucleotides of the AUG site. Alternatively, or in combination, the target binding sequence may be designed to hybridise to the target mRNA sequence downstream of said AUG site. In one embodiment, the sequence is reverse complementary to 0 to 40 nucleotides, such as 0 to 4 nucleotides of the target mRNA sequence downstream of said AUG site.

In one embodiment, the target determinant sequence is at least 10 nucleotides long and comprises, from 3' to 5':
- a sequence reverse complementary to 0 to 50 nucleotides of the 5' untranslated region (5' UTR) and 0 to 40 nucleotides of the coding sequence (CDS) of the target mRNA sequence; or
- a sequence reverse complementary to 0 to 80 nucleotides of the region upstream of an AUG site (start codon) of the target mRNA and 0 to 40 nucleotides of the CDS of the target mRNA sequence downstream of said AUG site.

In one embodiment, the target determinant sequence is at least 14 nucleotides long and comprises, from 3' to 5':
- a sequence reverse complementary to 0 to 40 nucleotides of the 5' UTR and 0 to 32 nucleotides of the CDS of the target mRNA sequence; or
- a sequence reverse complementary to 0 to 70 nucleotides of the region upstream of an AUG site (start codon) of the target mRNA and 0 to 4 nucleotides of the CDS of the target mRNA sequence downstream of said AUG site.

### DNA molecules and vectors

According to a further aspect of the invention, there is provided a DNA molecule encoding any of the functional nucleic acid molecules disclosed herein. According to a further aspect of the invention, there is provided an expression vector comprising said DNA molecule.

Exemplary expression vectors are known in the art and may include, for example, plasmid vectors, viral vectors (for example adenovirus, adeno-associated virus, retrovirus or lentivirus vectors), phage vectors, cosmid vectors and the like. The choice of expression vector may be dependent upon the type of host cell to be used and the purpose of use. In particular the following plasmids have been used for efficient expression of functional nucleic acid molecules:
Mammalian expression plasmids:

| | |
|---|---|
| Plasmid Name: | pCDNA3.1 (-) |
| Expression: | CMV promoter BGH poly(A) terminator |
| Plasmid Name: | pDUAL-eGFPA (modified from peGFP-C2) |
| Expression: | H1 promoter BGH poly(A) terminator |

Viral vectors:

| | |
|---|---|
| Vector Name: | pAAV |
| Virus: | Adeno-Associated Virus |
| Expression: | CAG promoter / CMV enhancer SV40 late poly(A) terminator |
| Vector Name: | rcLV -TetOne-Puro |
| Virus: | Lentivirus (3rd generation) |
| Expression: | LTR-TREt (Tre-Tight) promoter (doxycycline-inducible expression) BGH poly(A) terminator |
| Vector Name: | pLPCX-link |
| Virus: | Retrovirus (3rd generation) |
| Expression: | CMV |

It should be noted that any promoter may be used in the vector and will work just as well as those mentioned above.

### Compositions and methods

The present invention also relates to compositions comprising the functional nucleic acid molecules or the DNA molecules described herein. The composition may comprise components which enable delivery of said functional nucleic acid molecules by viral vectors (AAV, lentivirus and the like) and non-viral vectors (nanoparticles, lipid particles and the like). Alternatively, the functional nucleic acid molecule of the invention may be administered as naked or unpackaged RNA.

The functional nucleic acid molecule may be administered as part of a composition, for example compositions comprising a suitable carrier. In certain embodiments, the carrier is selected based upon its ability to facilitate the transfection of a target cell with one or more functional nucleic acid molecules.

Therefore, according to a further aspect of the invention, there is provided a composition comprising the functional nucleic acid molecule described herein.

A suitable carrier may include any of the standard pharmaceutical carriers, vehicles, diluents or excipients known in the art and which are generally intended for use in facilitating the delivery of nucleic acids, such as RNA. Liposomes, exosomes, lipidic particles or nanoparticles are examples of suitable carriers that may be used for the delivery of RNA. In a preferred embodiment, the carrier or vehicle delivers its contents to the target cell such that the functional nucleic acid molecule is delivered to the appropriate subcellular compartment, such as the cytoplasm.

The functional nucleic acid molecules of the invention can enhance translation of the target gene of interest with no effect on mRNA quantities of the target gene. Therefore they can successfully be used as molecular tools to validate gene function in cells, as well as to implement the pipelines of recombinant protein production.

According to a further aspect of the invention, there is provided a method for increasing the protein synthesis efficiency of a target in a cell comprising administering the functional nucleic acid molecule or the composition described herein, to the cell. Preferably the cell is a mammalian cell, such as a human or a mouse cell.

Methods of the invention result in increased levels of target protein in a cell and therefore find use, for example, in methods of treatment for diseases which are associated with gene defects (i.e. reduced protein levels and/or loss-of-function mutations of the encoding gene). Methods of the invention find particular use in diseases caused by a quantitative decrease in the predetermined, normal protein level. Methods of the invention can be performed *in vitro, ex vivo* or *in vivo.*

The methods described herein may comprise transfecting into a cell the functional nucleic acid molecule, DNA molecule or expression vector as defined herein. The functional nucleic acid molecule, DNA molecule or expression vector may be administered to target cells using methods known in the art and include, for example, microinjection, lipofection, electroporation, using calcium phosphate, self-infection by the vector or transduction of a virus.

### Methods of preparing a functional nucleic acid molecules

According to a further aspect of the invention, there is provided a method of preparing a functional nucleic acid molecule for use in enhancing translation of a target protein comprising:
(i) analysing the mRNA sequence of the target protein for an RNA binding protein region and preparing a target determinant sequence comprising a sequence reverse complementary to said binding sequence; and
(ii) connecting the target determinant sequence prepared in step (i) to a regulatory sequence comprising a SINE B2 element or a functionally active fragment of a SINE B2 element, or an IRES sequence or an IRES derived sequence.

The RNA binding protein region may be as described herein. In particular, HNRNPK and PTBP1 have been identified as RNA binding proteins that are involved in enhancing translation of the target mRNA. Therefore, the RNA binding protein region is selected from an HNRNPK binding region and a PTBP1 binding region. The mRNA sequence of the target protein may also be analysed for RNA binding protein regions as described in Table 2.

This method may be used to identify alternative target determinant sequences (i.e. binding domains). Therefore, in one embodiment, the target determinant sequence is not located in the 5' untranslated region (5' UTR) and/or a region upstream of an AUG site (start codon, such as a start codon within the CDS) of the mRNA sequence of the target protein.

It will be understood that the embodiments described herein may be applied to all aspects of the invention, *i.e.* the embodiment described for the functional nucleic acid molecules may equally apply to the claimed methods and so forth.

The invention will now be illustrated with reference to the following non-limiting examples.

### EXAMPLES

### EXAMPLE 1 - Materials and Methods

### Cell culture

Human Embryonic Kidney (HEK) 293T/17 cells purchased from ATCC were maintained in DMEM, high glucose, GlutaMAX Supplement, pyruvate (Gibco) supplemented with 10% fetal bovine serum (Sigma) and 1% penicillin-streptomycin (Wako) at 37°C, 5% CO₂.

### Plasmid and constructs

The pEGFP-C2 vector (expression vector for EGFP) was purchased from Clontech. SINEUP-GFP in pcDNA3.1 (-) vector was described previously in Carrieri et al. (2012) Nature, 491: 454-457. SINEUP-SCR, SINEUP-ΔSB2, SINEUP-ΔAlu and SINEUP-GFP constructs were cloned into the pCS2+ vector (Indrieri et al. (2016) Scientific reports, 6: 27315). SINEUP-ΔPTBP1 binding regions (Δ①-⑤) and SINEUP-ΔHNRNPK binding regions (Δ⑥-⑨) were designed with deletion of the binding sites from SINEUP-GFP. The BD (-34/+10) of SINEUP-UCHL1 is designed to replace the BD of SINEUP-GFP.

### Plasmid transfection and conditions

The pEGFP-C2 and SINEUP vectors were co-transfected into HEK293T/17 cells by using Lipofectamine 2000 (Invitrogen) with OptiMEM (1×) Reduced Serum Medium (Gibco). Testing EGFP mRNA translation at various time points after transfection confirmed that up-regulation of translation of EGFP mRNA occurred from 24 hours to 48 hours post-transfection. SINEUP-UCHL1 vector was transfected in HEK293T/17 cells as mentioned above and harvested after 48 hours of vector transfection.

### Measuring protein up-regulation by Western blotting assay.

Cells were plated in twelve-well plates, transfected with plasmid(s), lysed in Cell Lysis buffer (Cell Signaling), and incubated at 4°C for 1 hour. The cell lysates were applied to 10% precast polyacrylamide gels (Bio-Rad) for SDS-PAGE and transferred to nitrocellulose membranes (Amersham). The membranes were incubated for 1 hour at room temperature with the primary antibody, anti-GFP rabbit polyclonal antibody (1:1000 dilution; A6455, Thermo Fisher Scientific), and then for 45 minutes at room temperature with the secondary antibody, anti-rabbit IgG conjugated with HRP (P0448, Dako), and EGFP was detected by ECL detection reagent (Amersham). As a control, anti-β actin mouse monoclonal antibody (1:1000 dilution; A5441, Sigma Aldrich) was used as the primary antibody, and anti-mouse IgG-conjugated HRP (1:1000 dilution; P0447, Dako) was used as the secondary antibody. To detect endogenous target proteins, anti-hnRNPK mouse monoclonal antibody [3C2]-ChlP Grade (ab39975, Abcam), anti-PTBP1 mouse monoclonal antibody (32-4800, Thermo Fisher Scientific) and anti-UCHL1 mouse monoclonal antibody (CL3210, Sigma Aldrich) were used at 1:1000 dilution with overnight incubation at 4°C.

### RNA extraction and reverse transcription real-time quantitative PCR (RT-qPCR)

RNA was extracted using the RNeasy mini kit (Qiagen) following the manufacturer's instructions. For separation of nuclear and cytoplasmic fractions, the PARIS kit (ThermoFisher) was used to obtain nucleic and cytoplasmic fractionated lysate. TURBO DNA-free Kit (Invitrogen) was used for DNase I treatment to remove plasmid DNAs. For RT-qPCR, cDNA was synthesized using the PrimeScript 1st strand cDNA synthesis kit (Takara), and PCR was performed with SYBR Premix Ex Taq II (Takara) and the 7900HT Fast Real-Time PCR System (Applied Biosystems). The thermocycling protocol was 95°C for 30 s followed by 40 cycles of 95°C for 5 s and 60°C for 30 s. The RNA expression level was normalized to the level of GAPDH mRNA in each fraction. The primer sequence of SINEUP RNAs and GFP mRNAs was described in Zucchelli et al. (2015) Frontiers in cellular neuroscience, 9: 174.

### RNA FISH

FISH probes for target transcripts were designed using Stellaris RNA FISH designer (Biosearch Technologies), and fluorescently labelled with Quasar 570 (for SINEUP RNAs) or Quasar 670 (for EGFP mRNA and UCHL1 mRNA). Stellaris FISH Probes, Human GAPDH with Quasar 570 Dye (SMF-2026-1, Biosearch Technologies) were used as a positive control for FISH assessment. Cells were fixed with 4% paraformaldehyde (Wako) and permeabilized with 0.5% Triton X-100 (Sigma) at room temperature for 5 minutes. Hybridization was performed overnight at 37°C. Nuclei were visualized by incubation with Hoechst 33342 (H3570, Thermo Fisher Scientific). After sequential washing steps, cell images were detected using a SP8-HyVolution confocal laser scanning microscope (Leica Microsystems) with a 63x/1.4 oil objective lens; the images were processed using HyD detectors with Huygens Essential software (Scientific Volume Imaging). The RNA signals in the images were counted with Icy Spot Detector (Olivo-Marin (2002) Pattern Recognition, 35: 1989-1996), and percentage co-localization was calculated with Icy Colocalization Studio (Lagache et al. (2015) Cytometry. Part A: the journal of the International Society for Analytical Cytology, 87: 568-579).

### Detection of SINEUP RNA binding proteins (RBPs)

The protocol for detecting SINEUP RBPs was based on the protocol for the Magna ChIRP RNA Interactome kit (Merck Millipore) with the modification of cross-linking with 300 mJ/cm2 of 254 nm UV light (CL-1000 Ultraviolet Cross Link, UVP). The cell pellet (2 × 10⁷ cells) was suspended with 2 mL of Lysis buffer (Cell Signaling) supplemented with Protease Inhibitor Cocktail Set III (Merck Millipore), mixed by rotation at 4°C for 30 minutes, and then sonicated for 8 cycles (ON for 30 s, OFF for 30 s) using a Picoruptor Sonicator (Diagenode). Each tube of lysate was incubated overnight at 37°C in hybridization buffer (750 mM NaCl, 50 mM Tris-HCI (pH 7.5), 1 mM EDTA) with additional 15% v/v formamide (Sigma), Phenylmethanesulfonyl Fluoride (PMSF) (Cell Signaling), Protease Inhibitor Cocktail Set III (Merck Millipore) and SUPERase In RNase Inhibitor (Thermo Fisher Scientific) just before use, and 100 pmol of probe. Each lysate was then incubated at 37°C for 30 minutes with washed MagCapture Tamavidin 2-REV magnetic beads (Wako), which improves specificity of capture of specific RBPs. After sequential washes, the bead samples were separated into two halves for protein and RNA extraction. Proteins were extracted as reported by Chu et al. (2015) Cell, 161: 404-16; the eluent was incubated in DNase/RNase solution (100 µg/mL RNase A, 0.1 U/µL RNase H, and 100 U/mL DNase I) at 37°C for 1 hour followed by acetone precipitation. The protein samples were digested with 10 ng/µL Sequencing Grade Modified Trypsin (V5111, Promega) overnight, and the resultant peptides were subjected to Liquid chromatography-tandem mass spectrometry (LC-MS/MS) at the Support Unit for Bio-Material Analysis, Research Resource Center, Brain Science Institute in Wako, Japan. Proteome Discover (version 1.4, Thermo Fisher Scientific) software with the MASCOT search engine (version 2.6.0, Matrix Science Limited) was used in the Swiss-Prot database. For RNA extraction, the beads were incubated with proteinase K at 55°C overnight, and then extracted with Trizol (Thermo Fisher Scientific) and chloroform (Wako). The eluent was treated with DNase I (Ambion), and the RNA expression level was quantified by RT-qPCR.

### Validation of SINEUP RBPs by siRNA-mediated knockdown

All siRNAs listed below were purchased from Thermo Fisher Scientific. DNAJC1 Silencer Select Pre-designed siRNA (ID: s34557), EEF1A1 Silencer Select Pre-designed siRNA (ID: s4479), EEF2 Silencer Select Pre-designed siRNA (ID: s4492), HNRNPK Silencer Select siRNA: Standard (ID: s6738 and ID: s6737), HNRNPM Silencer Select Pre-designed siRNA (ID: s9259), HNRNPU Silencer Select Pre-designed siRNA (ID: s6743), LMNB1 Silencer Select Pre-designed siRNA (ID: s8226), and PTBP1 Silencer Select Validated siRNA (ID: s11434, ID: s11435 and ID: s11436) were used for the knockdown experiments; 4390843 Silencer Select Negative Control #1 siRNA was used as the negative control. Twenty-four hours after the cells were plated, the target pre-designed siRNA was transfected by using Lipofectamine RNAiMAX (Invitrogen), and the cells were maintained in DMEM (1×) +GlutaMAX-1 (Gibco) supplemented with 10% fetal bovine serum (Sigma) without penicillin-streptomycin (Wako) at 37°C, 5% CO₂ for 24 hours. Following this, the vectors were transfected into the cells as described above. Targeted proteins were detected by using the following anti-mouse monoclonal antibodies purchased from Santa Cruz: DnaJC1[D-10] (sc-514244), EF-1 α1 [CBP-KK1] (sc-21758), EF-2 [C-9] (sc-166415), hnRNP K/J [3C2] (sc-32307), hnRNP M [A-12] (sc-515008), hnRNP I [SH54] (sc-56701), hnRNP U [3G6] (sc-32315), and Lamin B1 [8D1] (sc-56144). The above primary antibodies were diluted 1 in 500 and then incubated at 4°C overnight. HRP-conjugated anti-mouse IgG (P0447, Dako) was used as secondary antibody diluted 1 in 1000 and then incubated at room temperature for 45 minutes for protein visualization.

### RNA immunoprecipitation (RIP) with SINEUP RBPs

RIP was performed using the Abcam protocol with some modifications. HEK293T/17 cells were plated into 10 cm plates, followed by plasmid transfection described above. On the following day, the cells were irradiated with 300 mJ/cm² of 254 nm UV light, and nuclei and cytoplasmic fractions were isolated. Nuclear pellets were sheared by sonication with 5 cycles (ON for 30 s, OFF for 30 s) using a Bioruptor Pico device. To immunoprecipitate RNA with the antibodies for target proteins, each lysate was incubated at 4°C overnight. Protein A/G magnetic beads (Invitrogen) were added to bind the target antibodies, and sequential washing was conducted to remove unbound antibodies. The anti-hnRNP K mouse monoclonal antibody [3C2]-ChlP Grade (ab39975, Abcam) and anti-PTBP1 mouse monoclonal antibody (32-4800, Thermo Fisher Scientific) were used to immunoprecipitate HNRNPK and PTBP1, respectively, in each nuclear or cytoplasmic fraction. To purify the RNA, the lysates were incubated with protease K at 55°C overnight, followed by Trizol (Thermo Fisher Scientific) and chloroform (Wako) extraction. RNA levels were quantified by RT-qPCR.

### Clone overexpression of SINEUP RBPs

Clone vectors, hnRNPK in pCMV6-XL5, and PTBP1 in pCMV6-AC, were purchased from Origene. After the cells were plated for 18 hours, target protein clone vectors were transfected using Lipofectamine 2000 (Invitrogen). The transfected cells were maintained in DMEM (1×) +GlutaMAX-1 (Gibco) supplemented with 10% fetal bovine serum (Sigma) without penicillin-streptomycin (Wako) at 37°C, 5% CO2 for 6 h. Following this, pEGFP-C2 and SINEUP vectors were transfected into the cells as described above.

### Immunofluorescence microscopy

HEK293T/17 were prepared as described above for RNA FISH. After the cells were permeabilized, the primary antibodies, anti-hnRNPK mouse monoclonal antibody [3C2]-ChlP Grade (ab39975, Abcam) and anti-PTBP1 mouse monoclonal antibody (32-4800, Thermo Fisher Scientific), were added and hybridized overnight. Alexa Fluor 647-conjugated goat anti-mouse IgG secondary antibodies (A-21236, Thermo Fisher Scientific) were used to visualize the results. Several organelle marker antibodies were tested as follows; anti-SC35 (S4045, Sigma-Aldrich), anti-nmt55/p54nrb (NONO, ab70335, Abcam), anti-ILF3 (ab133354, Abcam), anti-EF1A (sc-21758, SantaCruz), anti-ATP5 (mitochondria, ab14748, Abcam), anti-Calnexin (endoplasmic reticulum, ab202572, Abcam) and J2 monoclonal antibody (dsRNA, 10010200, SCICONS). MALAT1 probes labeled with Atto 633.

### Polysome fractionation

Polysome fractionation was performed as reported previously (Faye et al. (2014) J. Vis. Exp. 92: e52295). Briefly, 2.5 × 10⁶ cells were plated into 10 cm plates, followed by hnRNPK and PTBP1 clone vector transfection after 18 hours, and EGFP and SINEUP vector transfection 6 hours later. All transfections were performed as described above. The transfected cells were maintained in growth media without antibiotics for 48 hours following clone vector transfection, then incubated with 0.1 mg/mL cycloheximide for 5 minutes at 37°C followed by washing with ice-cold PBS containing 0.1 mg/mL cycloheximide. The harvested cells were centrifuged at 300 × g for 10 minutes at 4°C. The cell pellets were suspended with 200 µL of ice-cold lysis buffer (50 mM Tris-HCI pH 7.5, 100 mM NaCl, 30 mM MgCl₂, 0.1 mg/mL cycloheximide, 0.1% NP-40, with fresh RNase inhibitor and Proteinase inhibitor cocktail added just before use). The cell lysate was incubated for 10 minutes on ice followed by centrifugation at 2,000 × g at 4°C for 5 minutes to separate the nuclei. The cytoplasmic fraction was subjected to further centrifugation at 17,000 × g at 4°C for 5 minutes to remove cell debris. The cytoplasmic lysate was layered onto a 15%-45% sucrose gradient and centrifuged in an SW41Ti Beckman rotor at 190,000 × g at 4°C for 3.5 hours. The sucrose gradient was separated into 12 fractions calculated by Triax flow cell (Biocomp). Half of each fraction was treated with Proteinase K at 55°C overnight then followed by Trizol and chloroform extractions as described above. The eluent was treated with DNase I (Ambion), and the RNA expression level was quantified by RT-qPCR.

Using the other half of each fraction, proteins were isolated using the Thermo Fisher Scientific acetone precipitation protocol: 4 volumes of cold acetone were added to each sample, and then the samples were incubated at -20°C for 1 hour or overnight, and centrifuged at 13,000 × g for 10 minutes at 4°C. Each pellet was suspended in PBS and subjected to Western blotting analysis.

### Protein-protein direct interaction with chemical cross-linking

BS3 (bis [sulfosuccinimidyl] suberate) cross-linking was performed based on a published protocol (de la Parra et al. (2018) Nature Communications, 9: 3068). The cells were incubated with 0.6 mM BS3 (Thermo Fisher Scientific) for 30 minutes; the reaction was quenched by incubation with 1M Tris-HCl (pH 7.5) for 15 minutes at room temperature. The cell lysate was incubated with target antibodies as described for RIP. After sequential washes, the beads were incubated with 2 × Laemmli sample buffer (Bio-Rad) at 95°C for 20 minutes to dissociate proteins, followed by Western blotting assay.

### Double strand RNA (dsRNA) immunoprecipitation with chemical cross-linking

Formaldehyde cross-linking was performed based on the Abcam X-ChlP protocol, and nuclei and cytoplasmic fractions were isolated using the PARIS kit (ThermoFisher). To immunoprecipitate dsRNA with J2 monoclonal antibody (10010200, Scicons), fractionated lysates were incubated at 4°C overnight. The beads were washed three times with 1x RIPA buffer at 37°C for 5 minutes, and nucleic fraction beads underwent two further DNase treatments incubated at 37°C for 30 min. RNA was extracted as mentioned in the RIP protocol and quantified by RT-qPCR.

### Detection of PTBP1 and HNRNPK binding regions on the SINEUP-GFP RNA

The seCLIP protocol described by Van Nostrand E.L. et al. ((2017) mRNA Processing: Methods and Protocols. Springer New York, New York, NY, pp. E1-E1) was performed to identify the binding regions of PTBP1 and HNRNPK on SINEUP-GFP RNA. Briefly, HEK293T/17 cells were plated into 10 cm plates, followed by plasmid transfection as described above. After 24 hours of transfection, the cells were irradiated with 400 mJ/cm² of 254 nm UV light. Next, for each sample, 2 × 10⁷ cells were lysed and treated with DNase and RNase I. Meanwhile, 10 µg anti-hnRNPK (ab39975, Abcam) or anti-PTBP1 (32-4800, Thermofisher) antibodies were coupled to 125 µL magnetic beads (Dynabeads M-280 Sheep Anti-mouse IgG). To capture RBP-RNA complexes on beads, the cell lysate was incubated with a magnetic beads-coupled antibody at 4°C overnight. Next, 2% input was saved and RNA in IP samples was dephosphorylated and end treated with Poly Nucleotide Kinase followed by on-beads 3' RNA linker (with sample barcodes) ligation. After this, RBP-RNA complexes were detected by Western blotting and the region above 55 kDa was cut out from the nitrocellulose membrane blot. In order to isolate the RNA, membrane slices were treated with Proteinase K and Urea followed by acid phenol-chloroform extraction and clean-up by Zymo RNA kit (R1013). Further treatment of input and IP samples and cDNA library preparation steps are as described in the original protocol. Finally, the amplified library was purified using AMPure XP beads in 1:1.5 ratio and quantified using qPCR and Bioanalyzer High-sensitivity DNA chip. Libraries were sequenced on Illumina MiSeq platform (50 cycles for HNRNPK and 150 cycles for PTBP1, single-read).

After MiSeq sequencing, reads were adapter trimmed using cutadapt (version 2.7) and reads less than 18 bp in length were discarded. The libraries were then demultiplexed using an in-house program (splitByBarcode) based on a 6-mer sample barcode, and this barcode was stripped using fastx_trimmer (FASTX Toolkit version 0.0.14). Mapping was then performed against custom genome annotations with STAR (version 2.5.0a). Each file was mapped to an annotation consisting of the full human genome (hg38) plus an additional sequence for the SINEUP-GFP construct. To retain multiple mapping reads we opted to keep all primary alignments regardless of how many secondary alignments could be found for the same read, rather than keeping only the uniquely mapping reads. The mapped reads for the two replicates of the HNRNPK seCLIP and input samples were merged at this stage using the bamtools merge function (bamtools version 2.4.1).

SINEUP-GFP deletion mutants lacking PTBP1 or HNRNPK binding regions were synthesized by the commercial preparation service at GENEWIZ (Saitama, Japan). HEK293T/17 cells were prepared as described above (see cell culture and plasmid transfection), and EGFP up-regulation was measured by Western blotting assay (see Measuring protein up-regulation by Western blotting assay).

### Gene ontology (GO) enrichment analysis

The RNA-seq data (Lubelsky & Ulitsky (2018) Nature, 555: 107-111) discussed in this section have been obtained from the Sequence Read Archive (SRA) and are accessible through the SRA accession number SRP111756 (https://www.ncbi.nlm.nih.gov/sra/?term=SRP111756). Raw FASTQ files for MCF7 (paired end) were downloaded and processed to remove rRNA reads, check overall quality and remove any orphan reads from the paired end samples. Resultant files were mapped to the human genome (hg38) using STAR. Gene counts were generated using htseq-count, after prior filtering of secondary mapped and unmapped alignments. Count files were used in DESeq2 to generate a list of differentially expressed genes between control and siRNA knockdown for each cell fraction (cytoplasm, nucleus, whole cell extract). Genes with an FDR adjusted p-value of less than 0.05 were used for GO term enrichment analysis using the Bioconductor package topGO. The background for each analysis was the full list of genes with non-zero expression in that fraction. All GO terms had to have at least 10 annotated genes to be considered, and enrichment was tested using the Kolmogorov-Smirnov elim method, with a score of less than 0.05 considered significant.

### Statistical analysis

Statistical differences were measured using a paired, two-sided Student's t-test. Bar graphs were described as mean ± standard deviation (SD) from at least 3 independent experiments. Statistically significant changes relative to a negative control were represented with *p < 0.05, **p < 0.01. To test normality of all data sets, Kolmogorov-Smirnov test was used.

### EXAMPLE 2 - SINEUP-GFP enhances EGFP mRNA translation

To confirm the translational up-regulation activity of SINEUP constructs, the inventors produced synthetic SINEUPs targeting EGFP mRNA (Figure 1A). It was previously reported that SINEUP-GFP enhances EGFP levels more efficiently when cloned into a pCS2+ plasmid (Indrieri et al. (2016) Scientific reports, 6: 27315) than when cloned into a pcDNA3.1 plasmid (Zucchelli et al. (2015) Frontiers in cellular neuroscience, 9: 174; and Patrucco et al. (2015) Gene, 569: 287-293.). The expression levels of RNA produced by different plasmids often differ due to different promoters, stability, and polyadenylation status, and in the current study it was found that the level of SINEUP RNA transcripts (measured as copy number per cell) was approximately 1.5-fold higher for pCS2+ than for pcDNA3.1. Therefore, pCS2+ was used for all subsequent experiments. EGFP up-regulation activities of SINEUP-GFP was then examined using a series of deletion mutants in HEK293T/17 cells; a binding domain (BD) mutant, with a scramble BD sequence (SINEUP-SCR); an effector domain (ED) deletion mutant (SINEUP-ΔSB2), and an Alu element deletion mutant (SINEUP-ΔAlu) (Figure 1A). Consistent with previous studies, synthetic SINEUP-GFP in pCS2+ showed approximately 2-fold induction of EGFP levels compared to the no-insert control (vector containing no SINEUP construct) (Figure 1B, C). SINEUP-SCR and SINEUP-ΔSB2 did not significantly elevate the EGFP levels, but the Alu element deletion mutant (SINEUP-ΔAlu) and SINEUP-GFP enhanced EGFP levels, as expected. Because none of the constructs significantly affected the EGFP mRNA (Figure 1D), the results indicate that translation of EGFP mRNA was induced by SINEUP-GFP and SINEUP-ΔAlu, but not by SINEUP-SCR or SINEUP-ΔSB2.

### EXAMPLE 3 - SINEUP RNAs co-localized with EGFP mRNAs in the cytoplasm

A previous study showed that the natural SINEUP RNA (AS-Uchl1) is transported to the cytoplasm upon rapamycin treatment, enhancing Uchl1 mRNA translation (Carrieri et al. (2012) Nature, 491: 454-457). The inventors hypothesized that the subcellular distribution of SINEUP RNAs has a key role in regulating target mRNA translation. To elucidate the kinetic distribution of EGFP mRNA and SINEUP RNA, RNA FISH (fluorescence in situ hybridization) was performed following co-transfection of EGFP and SINEUP expression vectors (SINEUP-GFP or the deletion mutants) into HEK293T/17 cells. It was observed that EGFP mRNAs were predominantly localized in the cytoplasm (Figure 2A, d, i, n, s), whereas the SINEUP RNAs were distributed both in the nucleus and the cytoplasm (Figure 2A, c, h, m, r). In the nucleus, SINEUP RNAs were located throughout the nucleoplasm, but not in the nucleolus.

SINEUP RNAs formed intensively clustered spots, which were partially co-localized with several nuclear organelle markers. In the cytoplasm, SINEUP RNAs co-localized with EGFP mRNAs, appearing as numerous small dots distributed throughout the cytoplasm (Figure 2A, u, arrows). Co-localization of EGFP mRNA and SINEUP RNA in the cytoplasm was observed more frequently for SINEUP-GFP (37.60%) and SINEUP-ΔAlu RNAs (31.12%) than for SINEUP RNAs with impaired translational up-regulation activity (i.e., SINEUP-SCR and SINEUP-ΔSB2) (Figure 2B). This indicated that the BD and ED domains contribute both to the up-regulation of translation, and to the co-localization of EGFP mRNAs and SINEUP RNAs. When the EGFP expression vector was transfected alone, most EGFP mRNAs were distributed in the cytoplasm (data not shown), as was observed for co-transfection (Figure 2A, d, i, n, s). A similar cytoplasmic pattern was observed for EGFP mRNA when co-transfected with all SINEUP mutants (data not shown). In contrast, when the expression vectors for SINEUP RNAs were transfected alone, SINEUP RNAs were preferentially distributed in the nucleus (Figure 2C, e, h, k). To compare the subcellular distribution of SINEUP RNAs between the cells co-transfected with EGFP and SINEUP vectors to those transfected with SINEUP alone, the signals were detected in both the whole cell and the nuclear region alone using icy Spot Detector. The percentage of SINEUP-GFP RNA detected in the nucleus was 60.6% when the SINEUP-GFP vector was transfected alone (Figure 2D); this was significantly reduced to 49.3% when the SINEUP vector was co-transfected with the EGFP vector, meaning more SINEUP-GFP RNA was shifted to the cytoplasm. A similar finding was observed for SINEUP-ΔAlu, but no significant differences were observed for SINEUP-SCR or SINEUP-ΔSB2 (data not shown). As supported by qPCR measurements of RNA expression, subcellular distribution of SINEUP-ΔAlu and SINEUP-GFP RNA shifted to the cytoplasm when those transcripts were co-transfected with EGFP mRNA, while cytoplasmic SINEUP RNAs were reduced when the SINEUP vector was transfected alone (except for SINEUP-SCR) as compared to SINEUP vector with EGFP vector co-transfection. Consistent with these observations, translational up-regulation was enhanced by exporting the SINEUP RNAs into the cytoplasm in the presence of EGFP mRNA.

### EXAMPLE 4 - Identification and functional analysis of SINEUP RNA binding proteins

The inventors hypothesized that SINEUP RNA binding proteins (RBPs) may play a crucial role in EGFP expression. To identify SINEUP RBPs in the cells, a modified version of the Chromatin Isolation by RNA Purification (ChIRP) method (Chu et al. (2015) Cell, 161: 404-416) was used followed by mass spectrometry (MS) analysis. By carrying out three or more independent experiments on SINEUP-SCR, SINEUP-ΔSB2, SINEUP-ΔAlu and SINEUP-GFP transfection, several SINEUP RBPs were detected. To determine which RBPs are the most important for the translational up-regulation activity of SINEUP-GFP, several candidate SINEUP-GFP RBPs were selected with high reliability and specificity by the calculation of score from mass spectrometry database searched engine Mascot (see method, Figure 3A) while non-specific bound proteins, which were also detected in the samples with beads and labelled as LacZ probe, were removed to identify specific RBPs (Figure 3B). The SINEUP-GFP RBPs were then compared with those for the other SINEUP mutants (Figure 3C-E) and observed that several nucleocytoplasmic shuttling-related proteins were specifically enriched as SINEUP-GFP RBPs (Figure 3A). After excluding ribosomal proteins, siRNA-mediated knockdown of enriched proteins in the scatterplot (PTBP1, HNRNPK, DNAJC1, EEF2, EF1A1, HNRNPM, HNRNPU and LMNB1) was performed to assess their effects on the up-regulation of SINEUP-GFP protein translation (Figure 4). The experiments revealed that knockdown of either PTBP1 (Figure 4A, and Figure 5) or HNRNPK (Figure 4B, and Figure 5) significantly decreased the translational up-regulation activity of SINEUP-GFP. On the other hand, transfecting cells with scramble siRNA (negative control) did not affect the translational up-regulation activity of SINEUP-GFP (Figure 4J, SINEUP-GFP 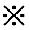 ,and Figure 5). Knockdown of EF1A1 decreased the up-regulation of translation for EGFP, but also affected β-actin levels as a non-specific effect (Figure 4F), suggesting it affected global translational pathways. Interestingly, knockdown of PTBP1 (Figure 6A, f-j) and HNRNPK (Figure 6A, k-o) significantly reduced the co-localization of EGFP mRNA and SINEUP-GFP RNA in the cytoplasm compared with the cells transfected by negative control siRNA; siRNA_Cont. (Figure 6A, a-e, and 6B). Furthermore, it significantly increased SINEUP-GFP RNA nuclear retention: 76.4% for PTBP1 knockdown and 61.4% for HNRNPK knockdown versus 49.7% in control cells (Figure 6C), suggesting a decrease in cytoplasmic SINEUP RNAs. In particular, knocking down PTBP1 significantly reduced cytoplasmic SINEUP RNAs in the cytoplasm (Figure 7A), while knockdown of HNRNPK reduced both SINEUP RNAs and EGFP mRNAs across the whole cell fraction (Figure 7A and B). These results suggest that SINEUP RNAs and EGFP mRNAs were not sufficient for SINEUP to function when HNRNPK levels are reduced after knockdown. The SINEUP RNA level in the whole cell lysate (WCL) following PTBP1 knockdown was not significantly changed, with reduction of SINEUP RNA only seen at the cytoplasmic level following PTBP1 knockdown. This suggests that PTBP1 does not have an effect on the transcript expression level and EGFP mRNA translation machineries, but instead has an effect on SINEUP RNA subcellular localization, consistent with changes in subcellular localization following PTBP1 overexpression (see Figure 8).

In the knockdown of HNRNPK, it was observed that the reduction of EGFP mRNA and SINEUP RNA co-localization resulted in the loss of EGFP enhancement. Since HNRNPK is a highly expressed protein related to several biological processes (Michael et al. (1997) EMBO J. 16: 3587-3598; Feliers et al. (2007) Am. J. Phys. Renal physiology, 293: F607-F615; and Naarmann et al. (2008) J. Biol. Chem. 283: 18461-18472), and associates with several target transcripts to contribute to their subcellular localization (Lubelsky & Ulitsky (2018) Nature, 555: 107-111), the inventors investigated whether the knockdown experiment has an effect on global translation. To do this published RNA-seq data from MCF7 cells was used with a knockdown timing similar to that employed in our experiment. As results, analysis of gene ontology (GO) terms after knockdown of HNRNPK in MCF7 cells suggested that translational regulation is not affected by perturbation of HNRNPK, as the number of genes involved in "translation" is negligible, while the cellular response seems to involve other processes (see Method section "GO enrichment analysis"). As a caveat, the experiment was performed in MCF7 cells, which may somehow differ from the HEK293T cells, although translation is generally a conserved cellular function. Therefore, PTBP1 and HNRNPK may mainly participate in the nucleocytoplasmic shuttling of SINEUP RNAs. To better understand the role of PTBP1 and HNRNPK interactions in SINEUP-GFP RNA nucleocytoplasmic shuttling, an RNA immunoprecipitation (RIP) assay was conducted of RNA-protein interactions with PTBP1 and HNRNPK proteins in the nucleus and cytoplasm. SINEUP-GFP RNAs were pulled down with PTBP1 both in the nucleus (Figure 9A1) and cytoplasm (Figure 9A2) and EGFP mRNAs were pulled down with HNRNPK both in the nucleus (Figure 9B1) and cytoplasm (Figure 9B2). These observations show that (a) PTBP1 protein was able to bind to SINEUP-GFP RNA or the EGFP-SINEUP RNA complex in either the nucleus or the cytoplasm, but not to EGFP mRNA alone; and (b) HNRNPK protein was able to bind to EGFP mRNA or the EGFP-SINEUP RNA complex in either the nucleus or the cytoplasm, but did not bind to SINEUP-GFP RNA alone. Taken together, these findings indicate that PTBP1 and HNRNPK play a role in the formation of the RNA-protein complexes and participate in the kinetic distribution of these RNA-protein complexes.

### EXAMPLE 5 - SINEUP RBPs drive subcellular localization of SINEUP RNAs and participate in translational initiation assembly

It was next considered whether EGFP levels would be further enhanced by overexpression of PTBP1 and HNRNPK proteins. To address this question, cells were transfected with either an PTBP1 or HNRNPK expression vector. The EGFP level was moderately, but significantly increased by overexpression of PTBP1 (Figure 10A1 and A3) or HNRNPK (Figure 10B1 and B3) in the cells co-transfected with EGFP and SINEUP-GFP vectors, but not in those transfected with EGFP vector alone (Figure 10A2 and B2). This finding suggests that PTBP1 and HNRNPK formed a protein-SINEUP RNA complex and functionally enhanced EGFP levels.

To evaluate the effect of SINEUP RBPs on the subcellular distribution of SINEUP RNA, PTBP1 (Figure 11A) or HNRNPK (Figure 11B) were overexpressed in the cells and then co-transfected with EGFP and SINEUP-GFP vectors, or with SINEUP-GFP vector alone. Some nuclear SINEUP-GFP RNAs were preferentially shuttled into the cytoplasm when EGFP and SINEUP-GFP transcripts were co-transfected into cells overexpressing PTBP1 compared to cells with normal PTBP1 levels (Figure 11A2: Figure 8A); this difference was not seen when the cells were transfected with SINEUP-GFP vector alone (Figure 11A3). Induction of PTBP1 did not directly drive SINEUP-GFP RNA from the nucleus to the cytoplasm without the presence of EGFP mRNAs. In contrast to PTBP1, overexpression of HNRNPK had no significant effect on the subcellular distribution of SINEUP-GFP RNAs (Figure 11B2 and B3; Figure 8A). Induction of PTBP1 or HNRNPK did not affect EGFP mRNA sub-cellular distribution (Figure 8B). Taking these results together, these findings indicate that PTBP1 and HNRNPK participate in nucleocytoplasmic shuttling of RNA-protein complexes and further act to regulate translation after shuttling into the cytoplasm.

To gain further insights into the molecular mechanism of translational enhancement, the distributions of the RNAs and RBPs in polysome fractions of cells overexpressing PTBP1 and HNRNPK was analysed. The cytoplasmic lysate was separated into 12 fractions using a 15%-45% sucrose gradient (Figure 12A). In co-transfected cells, EGFP mRNAs were slightly shifted into heavier polysomes in all cases (control, Figure 12B, d; PTBP1 overexpression, Figure 12B, e; HNRNPK overexpression, Figure 12B, f) compared with the corresponding cells when EGFP vector alone was transfected (control, Figure 12B, a; PTBP1 overexpression, Figure 12B, b; HNRNPK overexpression, Figure 12B, c). SINEUP-GFP RNA co-sedimented with EGFP mRNA in the heavy polysome fractions when EGFP mRNA was present (Figure 12B, g, h, i). Although it was observed in the FISH analysis (Figure 2C and D) that most SINEUP-GFP RNA was retained in the nucleus when the cells were transfected with SINEUP-GFP alone, more than 85% of the cytoplasmic SINEUP-GFP RNA sedimented in the fractions containing Free/40S binding RNAs (47.7%) and monosomes (37.8%) (Figure 12B, m). This implies that the cytoplasmic SINEUP-GFP RNA may participate in an initial phase of translation. Western blotting analysis of the co-distribution of SINEUP RBPs and RNAs was conducted in the polysome fractions (Figure 12B, j, k, l). The analysis revealed that HNRNPK co-sedimented with the RNAs and PTBP1 in the light polysome fractions when HNRNPK was overexpressed (Figure 12B, l). PTBP1 also co-sedimented with the RNAs in the light polysome fractions when PTBP1 was overexpressed (Figure 12B, k). Additionally, PTBP1 co-sedimented with SINEUP-GFP RNA from the Free/40S fractions and monosome fractions even when SINEUP-GFP vectors were transfected alone (Figure 12B, m). This indicates that SINEUP RNAs associate with PTBP1 and may recruit ribosome subunits to contribute to the formation of translational initiation complexes, including elongation factor EF1A, to participate in the initial phases of translation.

### EXAMPLE 6 - The SINEUP RBPs are crucial for enhancement of target mRNA translation and bind with several specific regions on SINEUP transcripts

To determine the specific binding sites of PTBP1 and HNRNPK on SINEUP-GFP RNA, seCLIP (single-end enhanced crosslinking and immunoprecipitation assay) was performed (Figure 13A). The binding regions are shown in Table 1.

**Table 1: Sequences of binding sites of HNRNPK and PTBP1 on SINEUP-GFP transcripts shown in Figure 13A**

| **Binding site** | | **Sequence** | **SEQ ID NO.** |
|---|---|---|---|
| HNRNPK binding regions | 1 | CCATGGTGGCGACCGGTAGCGCTAGCGGATCTG | 1 |
| | 2 | AAGAGGGCACAGCCACCGAGGATGGTACAGCATGGATGG ATGGTACGCTACAGAGACTCGGGAGCCCAACTGTGAGTG GCTGACTGGC | 2 |
| | 3 | GAGTGGGTCCTGGGCAAAGCAGGGGGTGCATCCCAGCCT CAGGGAATAGCACAGCAGAGGTCTGTTGATGCATGCGAG TGCATGACCTGCTTGCCAA | 3 |
| | 4 | TGAGTGGGAGAGGGGATGAGACATTCCTTTCTC | 4 |
| | 5 | ATTACTCTGTGTTGGCGGCGAGCTATCTGACAGCCAACCT TCCCATAC | 5 |
| PTBP1 binding regions | 6 | CTAGACTCGAGCCATGGTGGCGACCGGTAGCGCTAGCGG ATCTGACGGTTCACTAGATGCGGCCGCCACTGTGCTGGA TATCTGCAGAATTCGCCCTTCAGTGCTAGAGGAGGTCAGA | 6 |
| | 7 | CG | |
| | 8 | ATGAGCCTGGAAAGAGGGCACAGCCACCGAGGATGGTAC AGCATGGATGGATGGTACGCTACAGAGACTC | 7 |
| | 9 | AAGCATCATGGG | 8 |

The ability of binding region deletion mutants (SINEUP-ΔHNRNPK binding regions; Δ①-⑤, and SINEUP-ΔPTBP1 binding regions; Δ⑥-⑨, respectively) was then examined with different annealing sites (+a, +b, +c in Figure 13B) to up-regulate EGFP translation. It was found that all mutants, either lacking the sense-antisense region or those where the binding region was shifted outside the HNRNPK and PTBP1 binding regions (Δ①-⑤, Δ⑥-⑨), were ineffective to induce SINEUP activity (Figure 13C). The RNA level of EGFP mRNAs and SINEUP RNAs did not significantly change (Figure 13D), as expected. This shows that inhibition of PTBP1 and HNRNPK binding to SINEUP-GFP RNA at specific regions especially at surrounding BD results in the loss of EGFP up-regulation, therefore, the association of SINEUP-GFP RNA with PTBP1 and HNRNPK is crucial for its up-regulation activity.

### EXAMPLE 7 - The SINEUP RBPs are important for up-regulation of endogenous target mRNA translation.

To examine whether PTBP1 and HNRNPK are important for enhancement of endogenous target translation, SINEUP-UCHL1 were designed and transfected into HEK293T/17 cells. Consistent with the GFP studies, SINEUP-UCHL1 showed approximately 2-fold induction of UCHL1 protein levels compared with the No-insert control (vector without SINEUP) and SINEUP-SCR (Figure 14A). On the other hand, none of them significantly affect the Uchl1 mRNA level (Figure 14B). In order to investigate the distribution of Uchl1 mRNAs and SINEUP RNAs in the cells, RNA FISH was performed after the transfection of SINEUP expression vectors (No-insert, SINEUP-SCR and SINEUP-UCHL1) into HEK293T/17 cells. It was observed that Uchl1 mRNAs were mainly localized in the cytoplasm, whereas the SINEUP RNAs were distributed both in the nucleus and the cytoplasm (results confirmed by FISH). Consistent with the SINEUP-GFP studies, the co-localization of Uchl1 mRNA and SINEUP RNA in the cytoplasm was increased at the SINEUP-UCHL1 transfected cells compared with the SINEUP-SCR transfected cells (Figure 14C). When either PTBP1 (Figure 14D, a1, 2) or HNRNPK (Figure 14D, b1,2) was knocked down, the co-localization of Uchl1 mRNA and SINEUP RNA in the cytoplasm was decreased (Figure 14E), and resulted into the loss of translational up-regulation activity in the UCHL1 protein level compared with siRNA control cells (Figure 14D, c1, 2, SINEUP-UCHL1*). Note that the Uchl1 mRNA levels were not changed after the PTBP1 or HNRNPK knockdowns (data not shown). These results suggest that PTBP1 and HNRNPK are important factors of SINEUPs cellular network to enhance translational activity on both exogenous and endogenous target mRNAs.

**TABLE 2 - Example RNA binding protein regions**

| **Motif Sequence** | **Method** | **Cell line/ in vitro** | **Motif Source** |
|---|---|---|---|
| ***Example HNRNPK binding motifs*** | | | |
| ACCCAA | SELEX of 50nt random with recombinant protein. Consensus motif: u/aCCCCCu/a. Winners confirmed by EMSA with recombinant protein. | in vitro | ATtRACT |
| ACCCAT | SELEX of 50nt random with recombinant protein. Consensus motif: u/aCCCCCu/a. Winners confirmed by EMSA with recombinant protein. | in vitro | ATtRACT |
| ACCCCAA | SELEX of 50nt random with recombinant protein. Consensus motif: u/aCCCCCu/a. Winners confirmed by EMSA with recombinant protein. | in vitro | ATtRACT |
| ACCCCCAA | SELEX of 50nt random with recombinant protein. Consensus motif: u/aCCCCCu/a. Winners confirmed by EMSA with recombinant protein. | in vitro | ATtRACT |
| ACCCCCAC | SELEX of 50nt random with recombinant protein. Consensus motif: u/aCCCCCu/a. Winners confirmed by EMSA with recombinant protein. | in vitro | ATtRACT |
| ACCCCCCCCCTA | SELEX of 50nt random with recombinant protein. Consensus motif: u/aCCCCCu/a. Winners confirmed by EMSA with recombinant protein. | in vitro | ATtRACT |
| ACCCGA | SELEX of 50nt random with recombinant protein. Consensus motif: u/aCCCCCu/a. Winners confirmed by EMSA with recombinant protein. | in vitro | ATtRACT |
| ACCCGC | SELEX of 50nt random with recombinant protein. Consensus motif: u/aCCCCCu/a. Winners confirmed by EMSA with recombinant protein. | in vitro | ATtRACT |
| ACCCGT | SELEX of 50nt random with recombinant protein. Consensus motif: u/aCCCCCu/a. Winners confirmed by EMSA with recombinant protein. | in vitro | ATtRACT |

| ACCCTT | SELEX of 50nt random with recombinant protein. Consensus motif: u/aCCCCCu/a. Winners confirmed by EMSA with recombinant protein. | in vitro | ATtRACT |
|---|---|---|---|
| ACTTCCC | eCLIP | K562 | mCrossBase/eCLIP |
| AGCCCA | RNA Bind-n-Seq (RBNS) | in vitro | Dominguez *et al.* |
| AGCCCAA | RNA Bind-n-Seq (RBNS) | in vitro | Dominguez *et al.* |
| AGTCCCC | eCLIP | HepG2/K562 | mCrossBase/eCLIP |
| ATCCCAT | eCLIP | K562 | mCrossBase/eCLIP |
| ATCCCTC | eCLIP | HepG2/K562 | mCrossBase/eCLIP |
| ATGTCCC | eCLIP | K562 | mCrossBase/eCLIP |
| CAAGCCC | RNA Bind-n-Seq (RBNS) | in vitro | Dominquez *et al.* |
| CACACAC | RNA Bind-n-Seq (RBNS) | in vitro | Dominguez *et al.* |
| CACCAC | RNA Bind-n-Seq (RBNS) | in vitro | Dominquez *et al.* |
| CACCACC | RNA Bind-n-Seq (RBNS) | in vitro | Dominguez *et al.* |
| CACGC | RNA Bind-n-Seq (RBNS) | in vitro | Dominguez *et al.* |
| CACGCA | RNA Bind-n-Seq (RBNS) | in vitro | Dominguez *et al.* |
| CACGCAC | RNA Bind-n-Seq (RBNS) | in vitro | Dominguez *et al.* |
| CACGCC | RNA Bind-n-Seq (RBNS) | in vitro | Dominguez *et al.* |
| CACGCCA | RNA Bind-n-Seq (RBNS) | in vitro | Dominguez *et al.* |
| CACGCCC | RNA Bind-n-Seq (RBNS) | in vitro | Dominquez *et al.* |
| CACGTCC | RNA Bind-n-Seq (RBNS) | in vitro | Dominguez *et al.* |
| CACTTCC | eCLIP | K562 | mCrossBase/eCLIP |
| CAGCCC | RNA Bind-n-Seq (RBNS) | in vitro | Dominguez *et al.* |
| CAGCCCA | RNA Bind-n-Seq (RBNS) | in vitro | Dominguez *et al.* |
| CAGCCCC | RNA Bind-n-Seq (RBNS) | in vitro | Dominguez *et al.* |
| CAGTCCA | RNA Bind-n-Seq (RBNS) | in vitro | Dominguez *et al.* |
| CATCACC | RNA Bind-n-Seq (RBNS) | in vitro | Dominguez *et al.* |
| CATCCCT | eCLIP | K562 | mCrossBase/eCLIP |
| CATGCC | RNA Bind-n-Seq (RBNS) | in vitro | Dominquez *et al.* |
| CATGCCA | RNA Bind-n-Seq (RBNS) | in vitro | Dominguez *et al.* |
| CATGCCC | RNA Bind-n-Seq (RBNS) | in vitro | Dominguez *et al.* |
| CATGTCC | RNA Bind-n-Seq (RBNS) | in vitro | Dominguez *et al.* |
| CCAAACC | RNAcompete | in vitro | ATtRACT |
| CCAACCC | RNAcompete | in vitro | ATtRACT |
| CCACACC | RNA Bind-n-Seq (RBNS) | in vitro | Dominguez *et al.* |
| CCACACG | RNA Bind-n-Seq (RBNS) | in vitro | Dominquez *et al.* |
| CCACGC | RNA Bind-n-Seq (RBNS) | in vitro | Dominguez *et al.* |
| CCACGCA | RNA Bind-n-Seq (RBNS) | in vitro | Dominguez *et al.* |
| CCAGCCC | RNA Bind-n-Seq (RBNS) | in vitro | Dominguez *et al.* |
| CCAGTCC | RNA Bind-n-Seq (RBNS) | in vitro | Dominguez *et al.* |
| CCATACC | RNA Bind-n-Seq (RBNS) | in vitro | Dominguez *et al.* |
| CCATCAC | RNA Bind-n-Seq (RBNS) | in vitro | Dominguez *et al.* |
| CCATCC | RNA Bind-n-Seq (RBNS) | in vitro | Dominguez *et al.* |
| CCATCCC | eCLIP | HepG2 | mCrossBase/eCLIP |
| CCATGCC | RNA Bind-n-Seq (RBNS) | in vitro | Dominguez *et al.* |
| CCATTCC | RNA Bind-n-Seq (RBNS) | in vitro | Dominguez *et al.* |
| CCCAGTC | eCLIP | HeG2/K562 | mCrossBase/eCLIP |
| CCCATCC | eCLIP | HepG2/K562 | mCrossBase/eCLIP |
| CCCCAGT | eCLIP | HepG2 | mCrossBase/eCLIP |
| CCCCATC | eCLIP | HepG2 | mCrossBase/eCLI P |
| CCCCCCC | Homopolymer binding assay in HeLa nuclear extract | in vitro | ATtRACT |
| CCCGTCC | eCLIP | HepG2 | mCrossBase/eCLIP |
| CCGCCC | RNA Bind-n-Seq (RBNS) | in vitro | Dominguez *et al.* |
| CCGTCCA | RNA Bind-n-Seq (RBNS) | in vitro | Dominquez *et al.* |
| CCGTCCC | RNA Bind-n-Seq (RBNS) | in vitro | Dominguez *et al.* |
| CCTGCCC | RNA Bind-n-Seq (RBNS) | in vitro | Dominguez *et al.* |
| CCTGTCC | RNA Bind-n-Seq (RBNS) | in vitro | Dominguez *et al.* |
| CCTTACC | RNA Bind-n-Seq (RBNS) | in vitro | Dominguez *et al.* |
| CCTTGCC | RNA Bind-n-Seq (RBNS) | in vitro | Dominguez *et al.* |
| CGCCATC | RNA Bind-n-Seq (RBNS) | in vitro | Dominguez *et al.* |
| CGCGCCC | RNA Bind-n-Seq (RBNS) | in vitro | Dominguez *et al.* |
| CGCGTCC | RNA Bind-n-Seq (RBNS) | in vitro | Dominguez *et al.* |
| CGCTGCC | RNA Bind-n-Seq (RBNS) | in vitro | Dominquez *et al.* |
| CGTCACC | RNA Bind-n-Seq (RBNS) | in vitro | Dominguez *et al.* |
| CGTGCC | RNA Bind-n-Seq (RBNS) | in vitro | Dominguez *et al.* |
| CGTGCCC | RNA Bind-n-Seq (RBNS) | in vitro | Dominguez *et al.* |
| CGTGTCC | RNA Bind-n-Seq (RBNS) | in vitro | Dominguez *et al.* |
| CTAGCCC | RNA Bind-n-Seq (RBNS) | in vitro | Dominquez *et al.* |
| GCCACGC | RNA Bind-n-Seq (RBNS) | in vitro | Dominguez *et al.* |
| GCCCA | RNA Bind-n-Seq (RBNS) | in vitro | Dominguez *et al.* |
| GCCCAA | SELEX of 50nt random with recombinant protein. Consensus motif: u/aCCCCCu/a. Winners confirmed by EMSA with recombinant protein. | in vitro | ATtRACT |
| GCCCAAC | RNA Bind-n-Seq (RBNS) | in vitro | Dominguez *et al.* |
| GCCCAC | SELEX of 50nt random with recombinant protein. Consensus motif: u/aCCCCCu/a. Winners confirmed by EMSA with recombinant protein. | in vitro | ATtRACT |
| GCCCAG | SELEX of 50nt random with recombinant protein. Consensus motif: u/aCCCCCu/a. Winners confirmed by EMSA with recombinant protein. | in vitro | ATtRACT |
| GCCCCCCTA | SELEX of 50nt random with recombinant protein. Consensus motif: u/aCCCCCu/a. Winners confirmed by EMSA with recombinant protein. | in vitro | ATtRACT |
| GCCCCTA | SELEX of 50nt random with recombinant protein. Consensus motif: u/aCCCCCu/a. Winners confirmed by EMSA with recombinant protein. | in vitro | ATtRACT |
| GCGTGCC | RNA Bind-n-Seq (RBNS) | in vitro | Dominquez *et al.* |
| GGGGGGG | Western blot of AMA (human amnion) cell extracts and homoribopolymers. Western blot of recombinant proteins and homoribopolymers. | in vitro | ATtRACT |
| GTCCCCA | eCLIP | HepG2 | mCrossBase/eCLIP |
| TCCCAA | SELEX of 50nt random with recombinant protein. Consensus motif: u/aCCCCCu/a. Winners confirmed by EMSA with recombinant protein. | in vitro | ATtRACT |
| TCCCAC | SELEX of 50nt random with recombinant protein. Consensus motif: u/aCCCCCu/a. Winners confirmed by EMSA with recombinant protein. | in vitro | ATtRACT |
| TCCCAT | SELEX of 50nt random with recombinant protein. Consensus motif: u/aCCCCCu/a. Winners confirmed by EMSA with recombinant protein. | in vitro | ATtRACT |
| TCCCATC | eCLIP | HepG2/K562 | mCrossBase/eCLIP |
| TCCCCAA | SELEX of 50nt random with recombinant protein. Consensus motif: u/aCCCCCu/a. Winners confirmed by EMSA with recombinant protein. | in vitro | ATtRACT |
| TCCCCAT | SELEX of 50nt random with recombinant protein. Consensus motif: u/aCCCCCu/a. Winners confirmed by EMSA with recombinant protein. | in vitro | ATtRACT |
| TCCCCCAA | EMSA; UV cross-linking and immunoprecipitation with HT1080 cytoplasmic extracts or recombinant protein. | | ATtRACT |
| TCCCCCAT | SELEX of 50nt random with recombinant protein. Consensus motif: u/aCCCCCu/a. Winners confirmed by EMSA with recombinant protein. | in vitro | ATtRACT |
| TCCCCCCAA | EMSA; UV cross-linking and immunoprecipitation with HT1080 cytoplasmic extracts or recombinant protein. | | ATtRACT |
| TCCCCTA | SELEX of 50nt random with recombinant protein. Consensus motif: u/aCCCCCu/a. Winners confirmed by EMSA with recombinant protein. | in vitro | ATtRACT |
| TCCCCTT | SELEX of 50nt random with recombinant protein. Consensus motif: u/aCCCCCu/a. Winners confirmed by EMSA with recombinant protein. | in vitro | ATtRACT |
| TCCCGA | SELEX of 50nt random with recombinant protein. Consensus motif: u/aCCCCCu/a. Winners confirmed by EMSA with recombinant protein. | in vitro | ATtRACT |
| TCCCTA | SELEX of 50nt random with recombinant protein. Consensus motif: u/aCCCCCu/a. Winners confirmed by EMSA with recombinant protein. | in vitro | ATtRACT |
| TCCCTT | SELEX of 50nt random with recombinant protein. Consensus motif: u/aCCCCCu/a. Winners confirmed by EMSA with recombinant protein. | in vitro | ATtRACT |
| TGCCCA | RNA Bind-n-Seq (RBNS) | in vitro | Dominguez *et al.* |
| TGCCCAT | RNA Bind-n-Seq (RBNS) | in vitro | Dominguez *et al.* |
| TTTTTT | Homopolymer binding assay with recombinant protein. | | ATtRACT |
| ***Example PTBP1 binding motifs*** | | | |
| ACTTTCT RNAcompete in vitro ATtRACT | | | |
| ACTTTTT | RNAcompete | in vitro | ATtRACT |
| ATCTTC | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| ATTTTCT | RNAcompete | in vitro | ATtRACT |
| ATTTTTT | RNAcompete | in vitro | ATtRACT |
| CCCCCCC | Fluorescence spectroscopy with recombinant protein. | in vitro | ATtRACT |
| CCTAA | NMR | in vitro | ATtRACT |
| CCTAC | NMR | in vitro | ATtRACT |
| CCTAG | NMR | in vitro | ATtRACT |
| CCTAT | NMR | in vitro | ATtRACT |
| CCTCA | NMR | in vitro | ATtRACT |
| CCTCC | NMR | in vitro | ATtRACT |
| CCTCG | NMR | in vitro | ATtRACT |
| CCTCT | NMR | in vitro | ATtRACT |
| CCTCTTT | Mutagenesis; in vivo splicing assay | | ATtRACT |
| CCTGA | NMR | in vitro | ATtRACT |
| CCTGC | NMR | in vitro | ATtRACT |
| CCTGG | NMR | in vitro | ATtRACT |
| CCTGT | NMR | in vitro | ATtRACT |
| CCTTA | NMR | in vitro | ATtRACT |
| CCTTC | NMR | in vitro | ATtRACT |
| CCTTCCTT | Mutational analysis | | ATtRACT |
| CCTTG | NMR | in vitro | ATtRACT |
| CCTTT | NMR | in vitro | ATtRACT |
| CCTTTCCC | EMSA; UV crosslink; SDS-PAGE with HeLa nuclear extracts. | | ATtRACT |
| CCTTTCT | eCLIP | HepG2/K562 | mCrossBase/eCLIP |
| CCTTTCT | RNAcompete | in vitro | ATtRACT |
| CCTTTTT | RNAcompete | in vitro | ATtRACT |
| CTAA | NMR | in vitro | ATtRACT |
| CTAC | NMR | in vitro | ATtRACT |
| CTAG | NMR | in vitro | ATtRACT |
| CTAT | NMR | in vitro | ATtRACT |
| CTATCCT | eCLIP | HepG2 | mCrossBase/eCLIP eCLIP data |
| CTCA | NMR | in vitro | ATtRACT |
| CTCC | NMR | in vitro | ATtRACT |
| CTCCATCTT | EMSA with recombinant protein. RNA footprinting. | | ATtRACT |
| CTCG | NMR | in vitro | ATtRACT |
| CTCT | SOLUTION NMR | in vitro | ATtRACT |
| CTCT | NMR | in vitro | ATtRACT |
| CTCTC | in vitro RNA pulldown | in vitro | ATtRACT |
| CTCTCT | Immunodepletion; UV crosslink in WERI-1 and HeLa extracts; immunoprecipitation; immunoblot; NMR. | | ATtRACT |
| CTCTTA | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| CTCTTC | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| CTCTTCT | eCLIP | K562 | mCrossBase/eCLIP |
| CTCTTG | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| CTCTTT | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| CTCTTTC | eCLIP | K562 | mCrossBase/eCLIP |
| CTGA | NMR | in vitro | ATtRACT |
| CTGC | NMR | in vitro | ATtRACT |
| CTGG | NMR | in vitro | ATtRACT |
| CTGT | NMR | in vitro | ATtRACT |
| CTTA | NMR | in vitro | ATtRACT |
| CTTC | NMR | in vitro | ATtRACT |
| CTTCTCTCT | UV-crosslink; EMSA | | ATtRACT |
| CTTG | NMR | in vitro | ATtRACT |
| CTTT | NMR | in vitro | ATtRACT |
| CTTTCCT | eCLIP | HepG2 | mCrossBase/eCLI P |
| CTTTCTC | eCLIP | HepG2/K562 | mCrossBase/eCLIP |
| CTTTCTT | RNAcompete using recombinant protein | in vitro | ATtRACT |
| CTTTTCT | RNAcompete | in vitro | ATtRACT |
| CTTTTTT | RNAcompete | in vitro | ATtRACT |
| GGCTCCCCC | In Vitro Splicing;Uv cross-linking;HeLa cell | | ATtRACT |
| GGGGGGG | Fluorescence spectroscopy with recombinant protein. | in vitro | ATtRACT |
| GTCTTA | SELEX of 20nt random with recombinant protein. Consensus motifs: TCTT and TCTTC. UV crosslink and SDS-PAGE with HeLa nuclear extract. Equilibrium binding assays with another protein or other RNAs as competitors. | | ATtRACT |
| GTCTTCT | eCLIP | HepG2 | mCrossBase/eCLIP |
| GTCTTT | UV crosslink and SDS-PAGE with HeLa nuclear extract. Equilibrium binding assays with another protein or other RNAs as competitors. | | ATtRACT |
| GTCTTT | Mutagenesis; Pull down assay and Western blot with HeLa nucelar extract. | in vitro | ATtRACT |
| TAGCTGTG | EMSA with recombinant protein. RNA footprinting. | | ATtRACT |
| TCCTCTTC | Chemical shift mapping (NMR) | in vitro | ATtRACT |
| TCCTCTTC | NMR | in vitro | ATtRACT |
| TCCTTTC | eCLIP | HepG2/K562 | mCrossBase/eCLIP |
| TCTAA | NMR | in vitro | ATtRACT |
| TCTAC | NMR | in vitro | ATtRACT |
| TCTAG | NMR | in vitro | ATtRACT |
| TCTAT | NMR | in vitro | ATtRACT |
| TCTATCT | RNAcompete using recombinant protein | in vitro | ATtRACT |
| TCTCA | NMR | in vitro | ATtRACT |
| TCTCC | NMR | in vitro | ATtRACT |
| TCTCG | NMR | in vitro | ATtRACT |
| TCTCT | NMR | in vitro | ATtRACT |
| TCTCTC | UV crosslink; western blot; immunoprecipitation in HeLa nuclear extracts. | | ATtRACT |
| TCTCTCT | eCLIP | HepG2/K562 | mCrossBase/eCLIP |
| TCTCTTC | eCLIP | HepG2/K562 | mCrossBase/eCLIP |
| TCTGA | NMR | in vitro | ATtRACT |
| TCTGC | NMR | in vitro | ATtRACT |
| TCTGG | NMR | in vitro | ATtRACT |
| TCTGT | NMR | in vitro | ATtRACT |
| TCTT | SELEX | in vitro | ATtRACT |
| TCTTA | NMR | in vitro | ATtRACT |
| TCTTC | NMR | in vitro | ATtRACT |
| TCTTCTC | eCLIP | HepG2/K562 | mCrossBase/eCLIP |
| TCTTCTCT | NMR | in vitro | ATtRACT |
| TCTTCTCTCTT | UV cross-link with recombinant protein and with HeLa nuclear extract containing increasing protein concentrations. Mutation analysis. | | ATtRACT |
| TCTTCTCTTC | NMR | in vitro | ATtRACT |
| TCTTCTT | eCLIP | HepG2/K562 | mCrossBase/eCLIP |
| TCTTG | NMR | in vitro | ATtRACT |
| TCTTT | Chemical shift mapping (NMR) | in vitro | ATtRACT |
| TCTTTCT | RNAcompete usinq recombinant protein | in vitro | ATtRACT |
| TCTTTTT | RNAcompete | in vitro | ATtRACT |
| TTACTTT | RNAcompete using recombinant protein | in vitro | ATtRACT |
| TTATTTTTCCCC | EMSA; UV crosslink; immunoprecipitation with recombinant protein. | | ATtRACT |
| TTCTCTTTTCT | EMSA with recombinant protein. | in vitro | ATtRACT |
| TTCTTC | In vivo splicing assay; Mutagenesis; UV cross-linking | | ATtRACT |
| TTCTTCT | eCLIP | K562 | |
| TTCTTG | SELEX of 20nt random with recombinant protein. Consensus motifs: TCTT and TCTTC. UV crosslink and SDS-PAGE with HeLa nuclear extract. Equilibrium binding assays with another protein or other RNAs as competitors | | ATtRACT |
| TTCTTT | Mutagenesis; Pull down assay and Western blot with HeLa nucelar extract. | in vitro | ATtRACT |
| TTCTTTT | In vivo splicing assay; Mutagenesis; UV cross-linking | | ATtRACT |
| TTTTTCCC | Gel mobility shift; Mutagenesis; UV cross-linking; Competition experiments | | ATtRACT |
| TTTTTCT | RNAcompete | in vitro | ATtRACT |
| TTTTTTT | Fluorescence spectroscopy with recombinant protein | in vitro | ATtRACT |

| | | | |
|---|---|---|---|
| Abbreviations. **ATtRACT:** A database of RNA binding proteins and associated motifs (see https://attract.cnic.es/); **Dominguez et al.:** Dominguez et al. (2018) Mol. Cell 70: 854-867; **eCLIP**: enhanced crosslinking and immunoprecipitation assay; **EMSA:** Electrophoretic Mobility Shift Assay; **mCrossBase/eCLIP**: Top consensus motifs analyzed by mCrossBase using ENCODE eCLIP data; **NMR:** Nuclear Magnetic Resonance; **SDS-PAGE:** sodium dodecyl sulphate-polyacrylamide gel electrophoresis; **SELEX:** Systematic evolution of ligands by exponential enrichment. | | | |

## Claims

1. A functional nucleic acid molecule comprising:
(a) at least one target determinant sequence comprising a sequence reverse complementary to a target mRNA sequence for which protein translation is to be enhanced;
(b) at least one sequence encoding an RNA binding protein region; and
(c) at least one regulatory sequence comprising a SINE B2 element or a functionally active fragment of a SINE B2 element or an internal ribosome entry site (IRES) sequence or an IRES derived sequence.

2. The functional nucleic acid molecule of claim 1, wherein the RNA binding protein region is selected from a HNRNPK binding region and a PTBP1 binding region, for example a binding region as described in Table 2.

3. The functional nucleic acid molecule of claim 2, wherein the HNRNPK binding region is selected from a sequence with at least 60% sequence identity to SEQ ID NO: 1-5.

4. The functional nucleic acid molecule of claim 2, wherein the PTBP1 binding region is selected from a sequence with at least 60% sequence identity to SEQ ID NO: 6-8.

5. The functional nucleic acid molecule of any one of claims 1 to 4, wherein the RNA binding protein region is located 5' of the target determinant sequence.

6. The functional nucleic acid molecule of any one of claims 1 to 5, wherein the regulatory sequence is located 3' of the target determinant sequence

7. The functional nucleic acid molecule of any one of claims 1 to 6, further comprising at least one linker sequence between the at least one target binding sequence and the at least one regulatory sequence.

8. The functional nucleic acid molecule according to any one of claims 1 to 7 wherein the target determinant sequence is at least 10 nucleotides long and comprises, from 3' to 5':
- a sequence reverse complementary to 0 to 50 nucleotides of the 5' untranslated region (5' UTR) and 0 to 40 nucleotides of the coding sequence (CDS) of the target mRNA sequence; or
- a sequence reverse complementary to 0 to 80 nucleotides of the region upstream of an AUG site (start codon) of the target mRNA and 0 to 40 nucleotides of the CDS of the target mRNA sequence downstream of said AUG site.

9. The functional nucleic acid molecule according to claim 8, wherein the target determinant sequence is at least 14 nucleotides long and comprises, from 3' to 5':
- a sequence reverse complementary to 0 to 40 nucleotides of the 5' UTR and 0 to 32 nucleotides of the CDS of the target mRNA sequence; or
- a sequence reverse complementary to 0 to 70 nucleotides of the region upstream of an AUG site (start codon) of the target mRNA and 0 to 4 nucleotides of the CDS of the target mRNA sequence downstream of said AUG site.

10. The functional nucleic acid molecule according to any one of claims 1 to 9, wherein the molecule is circular.

11. A DNA molecule encoding the functional nucleic acid molecule according to any one of claims 1 to 10.

12. An expression vector comprising the functional nucleic acid molecule according to any one of claims 1 to 10, or the DNA molecule according to claim 11.

13. A composition comprising the functional nucleic acid molecule according to any one of claims 1 to 10, the DNA molecule according to claim 11 or the expression vector according to claim 12.

14. A method for increasing the protein synthesis efficiency of a target in a cell comprising administering the functional nucleic acid molecule according to any one of claims 1 to 9 or the composition according the claim 10 to the cell.
